# EUROPEAN PATENT APPLICATION

(11) **EP 2 316 820 A1**
(43) Date of publication of application: **04.05.2011**
(21) Application number: 09174390.6
(22) Date of filing: 28.10.2009
(51) Int. Cl.: C07C 235/34, C07C 237/20, C07C 233/01, C07C 309/65, C07D 211/06, C07D 277/46, C07D 295/12, A61K 31/16, A61K 31/18, A61P 29/00, A61P 11/00, A61P 25/04

(54) **2-aryl-propionamide derivatives useful as bradykinin receptor antagonists and pharmaceutical compositions containing them**

(71) Applicant: Dompe S.p.A., 67100 L'Aquila (IT)
(72) Inventor: Beccari, Andrea, 67100, L`Aquila AQ (IT); Aramini, Andrea, 67100, L`Aquila AQ (IT); Bianchini, Gianluca, 67100, L`Aquila AQ (IT); Moriconi, Alessio, 67100, L`Aquila AQ (IT)
(74) Representative: Perani, Aurelio

(57) **Abstract**

(R,S) 2-aryl-propionamide derivatives, or their single enantiomers (R) and (S) are disclosed useful in the treatment or prevention of symptoms and disorders such as pain and inflammation associated with the bradykinin B1 pathway.

## Description

### Field of the invention

The present invention relates to (R,S) 2-aryl-propionamide derivatives, their single enantiomers (R) and (S) and to pharmaceutical compositions containing them, which are useful in the treatment or prevention of symptoms and disorders such as pain and inflammation associated with the bradykinin B1 pathway.

### Background of the invention

The nonapeptide bradykinin (BK) and the physiologically-related decapeptide kallidin (KD) are endogenous vasoactive peptides generated as short-lived components of the kallikrein-kinin system. They play a key role in the regulation of normal physiological processes in the peripheral (PNS) and central (CNS) nervous systems and are effectors of a number of inflammatory responses, including bronchoconstrition, plasma extravasation, release of prostaglandins and leukotrienes, smooth muscle contraction and relaxation and nociception [Austin C.E. et al., J. Biol. Chem. (1997) 272, 11420-11425; Hess J.F. et al. Biochem. Biophys. Res. Commun. (1992) 184, 260-268]. Under pathophysiological conditions, elevated levels of kinins are rapidly produced from the circulating precursors kininogens by enzymatic action of trypsine-like serine proteases, kallikrein and tissue kallikrein. Kinins exert their action interacting with two cell surface receptors, BKB1R and BKB2R, belonging to the 7TM-GPCR superfamily. Through the Gα_{q} protein subunit they stimulate the phospholipase C-dependent pathway to increase the intracellular free calcium concentration and inositol phosphate formation, while through the Gαᵢ subunit activation they inhibit adenylcyclase and, by consequence, the formation of cAMP. BKB2Rs are constitutively expressed in most cells and tissue types and mediate the most part of acute effects due to BK and KD after their production in plasma and tissues, respectively. BKB1Rs are poorly constitutively expressed under physiological conditions and are induced following inflammatory insults or noxious stimuli, although recent data show the presence of constitutive BKB1Rs in rat and mouse CNS, making BKB1R a particularly attractive drug target.

Overproduction of kinins under pathophysiological conditions is implicated in the pathogenesis of a number of clinically-relevant disorders, including pain, inflammation, hypotension, asthma, colitis, rhinitis, pancreatitis, sepsis and rheumatoid arthritis [Leeb-Lundberg L.M.F. et al., Pharmacol. Rev. (2005) 57, 57, 27-77]. BK is also implicated in peripheral inflammatory processes associated with Alzheimer's disease [Huang H.M. et al., J. Neurochem. (1995) 64, 761-766], in the growth of several solid tumors [Stewart J.M. Curr. Pharm. Design (2003) 9, 2036-2042] and is also thought to play a cardioprotective role [Heitsch H. Expert Opin. Investig. Drugs (2003) 12, 759-770] as evidenced by BKB2R antagonists in alleviating congestive heart failure, hypertension and ischemic heart disease. The putative role of kinins, specifically BK, in the management of pain and inflammation has been well documented [Marceau F. et al. Nat. Rev. Drug Discov. (2004) 3, 845-852] and has provided impetus to the development of potent and selective BK antagonists. The BKB1R is an attractive target to treat inflammation because it is absent in normal tissues in most systems but it is inducible following tissue injury under the control of inflammatory cytokines, mitogen-activated protein kinase (MAPK) pathways and some transcription factors such as nuclear factor κB (NF-κB). BKB1R is more resistant than BKB2R to desensitization [Marceau F. et al. Pharmacol. Rev. (1998) 50, 357-386] making BKB1R antagonism more adapted to chronic or persistent inflammatory systems than BKB2R antagonism, More, the proposed protective effect of kinins mediated by the endothelial BKB2Rs on microcirculation in ischemia, diabetes and other pathological situations is a potential concern limited to BKB2R antagonists. On these basis, several research programs, also by industrial organizations, have been developed for the identification of novel non-peptide ligands binding BKB1 receptors replacing classical peptide antagonists. In recent years these efforts have been heightened with the expectation that useful therapeutic agents with anti-inflammatory properties would provide relief from diseases mediated by a BK receptor pathway [Bock M.G. et al. Current Opinion in Chem. Biol. (2000) 4, 401-406].

Non peptide BKB 1 R antagonists have appeared in the literature since the year 2000 and several of the disclosed structures, generated by different laboratories and belonging to different chemical classes, seem to share a possible common pharmacophore determined by the presence of a common moiety "RN-SO₂-phenyl" [Marceau F. TRENDS Pharmacol. Sc. (2005) 26, 116-118] that has allowed to derive a hypothesis of docking to the human B1 receptor and suggests structural commonalities and a preferential molecular mode of action within the selected compounds.

Along the last few years several classes of non peptide BKB1R antagonists have been disclosed. Three main classes have been claimed by several pharmaceutical companies:
N-(Arylsulfonyl)aminoacid derivatives [Sanofi W09725315 (1997); Novartis WO 00075107 (2000) and WO02092556 (2002); Bayer AG WO03007958 (20039; Elan Pharmaceuticals WO03093245 (2003); Lab. Fournier SA FR2840897 (2003); Merck & Co. INC. WO2004/054584 (2004)];
Biaryl derivatives [pharmacopoeia Inc. WO0105783 (2001); Merck & Co. INC. US2004034064 (2004), US2004029920 (2004), US 2004063761 (2004)];
Benzodiazepine derivatives [Merck & Co. INC. WO02099388 (2002)].

We have now found out a novel class of 2-arylpropionamide derivatives selective B1 bradykinin antagonists. Said compounds are useful in the treatment of pathologies depending on the bradykinin pathways B1 receptor-dependent.

### Detailed description of the invention

The present invention provides (R,S)-2-aryl-propionamide derivatives of formula (I) and their single (R) and (S) enantiomers as well as their pharmaceutically acceptable salts: 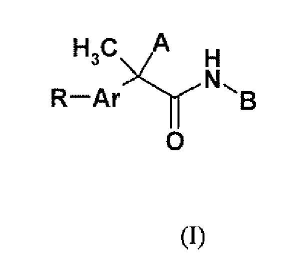 wherein
A is selected from the group consisting of H, CH3 and F;
Ar is a selected from the group consisting of phenyl and 5, 6-membered heteroaryl, said heteroaryl being preferably selected from tiophene and pyrrole;
R is a residue selected from the group consisting of:
- linear or branched C₁-C₆-alkyl or C₂-C₈-alkenyl, C₁-C₄-aminoalkyl,
- 3-6 membered cycloalkylamino;
- W-Ar₁ wherein W is selected from O, NH, CO and Ar₁ is selected from the group consisting of optionally substituted phenyl, naphtyl, quinolinyl, benzodioxolyl and 5-6-membered heteroaryl;
- optionally substituted 5-6-membered heterocyclic residues; and
- X-SO₂R,, wherein X is selected from NH and O and R1 is selected from linear or branched C₁-C₄ alkyl, C₁₋C₄ haloalkyl and optionally substituted phenyl;

B is a residue selected from the group consisting of:
- H, linear or branched C₁-C₆ alkyl, C₂-C₈-alkenyl, C₁-C₄ alkylamino, carbamoyl;
- (CH₂)ₙ-(NH)ₚ-Y wherein n is between 0 and 3, p is 0 or 1 and Y is selected from:
   - a 5-6 membered ring selected from optionally substituted phenyl, heteroaryl, cycloalkyl and heterocyclic residues;
   - benzyl, 5-6 membered heteroarylcarbonyl, C₁-C₆-alkyl, linear or branched C₁-C₃-alkylcarbonyl, C₁-C₆-alkoxy and C₁-C₆-alkoxy hydroxy substituted.
   - (CH₂)ₙ-Z-(CH2)_{n'}-A wherein n is between 0 and 3, n' is between 0 and 1, Z is selected from -CONH-, -O-, -NCH₃-, -CHOH- and A is selected from linear or branched C₁-C₄ alkyl, substituted or unsubstituted phenyl, substituted or unsubstituted phenoxy;
   - CHRₐR_{b}, wherein Rₐ and R_{b} are independently selected from substituted or unsubstituted 5-6 membered heteroaryl, substituted or unsubstituted 5-6 membered heterocyclic, substituted or unsubstituted phenyl, dialkylamino, -CH₂-NHCOO-C₁-C₄-alkyl, -(COO)C1-C₄-alkyl.

The term "substituted" in the above definitions means substituted by one or more groups independently selected from linear or branched C₁-C₅-alkyl, halogen, hydroxy, linear or branched C₁-C₅-alkoxy, linear or branched C₁-C₅-mercapto, halo-C₁-C₃-alkyl, halo-C₁-C₃-alkoxy, amino, C₁-C₅-alkylamino, linear or branched C₁-C₅-alkanesulfonamides.

In the particularly preferred compounds the term "substituted" means substituted by one or more groups independently selected from methyl, ethyl, isopropyl, tert-butyl, Cl, F, hydroxy, methoxy, thiomethyl, trifluoromethyl, trifluoromethoxy, isopropylsulfonyl, sulfonamido, amino.

According to a preferred embodiment of the invention, when Ar is phenyl, R in position 3 or 4 position of the aromatic ring.

R is preferably selected from the following residues: hex-1-en-1-yl, 2-methylpropyl, cyclopropylamino, substituted or unsubstituted phenylcarbonyl, substituted or unsubstituted tiophen-carbonyl, substituted or unsubstituted phenylamino, substituted or unsubstituted 1,3-thiazol-2-yl-amino, substituted or unsubstituted 1,3-oxazol-2-yl-amino, substituted or unsubstituted phenoxy, substituted or unsubstituted naphtalen-1-yloxy, substituted or unsubstituted naphtalen-2-yloxy morpholin-4-yl, pyperidin-1-yl, trifluoromethanesulfonyloxy, C1-C4 alkylsulfonylamino, substituted or unsubstituted phenylsulfonylamino, substituted or unsubstituted phenylsulfonyloxy.

B is preferably selected from H, ethyl, 2-methylprop-2-en-1-yl, 2-amino-2-methyl-propyl, substituted or unsubstituted 1*H*-pyrazol-4-yl, substituted or unsubstituted 1*H-*pyrazol-5-yl, substituted or unsubstituted tiophen-3-yl, substituted or unsubstituted 1,3-thiazol-2-yl, pyrimidin-4-yl, substituted or unsubstituted 1-*H-*pyrrol-1-yl, substituted or unsubstituted 4*H*-1,2,4-triazol-4-yl, substituted or unsubstituted pyridine-4-yl, pyrazin-2-yl, substituted or unsubstituted piperydin-4-yl, substituted or unsubstituted phenyl, substituted or unsubstituted ciclohexyl, furan-2-yl-C₁-C₃-alkyl, substituted or unsubstituted piperidin-1-yl-C₁-C₃-alkyl, pyridine-2-yl-amino-C1-C3-alkyl, phenylamino-C₁-C₃-alkyl, cyclohexylamino-N-C₁-C₃-alkyl, 1*H*-pyrazol-1-yl-C₁-C₃-alkyl, pyridin-4-yl-C₁-C₃-alkyl, morpholin-4-yl-C₁-C₃-alkyl, pyrrolidin-l-yl-C₁-C₃-alkyl, (C₁-C₆-alkylamino)-C₁-C₃-alkyl, (benzylamino)C₁-C₃-alkyl, (C₁-C₃-alkylamino)-ethyl, -(C₁-C₄-dialkylamino)C₁-C₃-alkyl, 2-(tert-butylamino)-2-oxoethyl; (phenoxy)C₁-C₃alkyl, [(benzyl)(methylamino)]C₁-C₃alkyl, (3,4-dimethylphenoxy)-2-, [(dimethylamino)(4-fluorophenyl)methyl]amino; (*tert-*butoxycarbonyl) aminoetylcarboxy], carbamoyl, furan-2-carbamido.

Particularly preferred compounds of the invention are:
4-(1-amino-2-fluoro-1-oxopropan-2-yl)phenyl trifluoromethanesulfonate 4-(2-fluoro-1-{[2-(5-methyl-1*H*-pyrazol-1-yl)ethyl]amino}-1-oxopropan-2-yl)phenyl trifluoromethanesulfonate
4-(2-fluoro-1-oxo-1-{[2-(pyridin-2-ylamino)ethyl]amino}propan-2-yl)phenyl trifluoromethanesulfonate
2-fluoro-*N*-(2-sulfamoylthiophen-3-yl)-2-(3-{[4-(trifluoromethyl)-1,3-thiazol-2-yl]amino}phenyl)propanamide
2-fluoro-*N*-(2-sulfamoylphenyl)-2-(3-{[4-(trifluoromethyl)-1,3-thiazol-2-yl]amino} phenyl)propanamide
4-(2-methyl-1-{[2-(*tert*-butylamino)-2-oxoethyl]amino}-1-oxopropan-2-yl)phenyl trifluoromethanesulfonate
4-(2-methyl-1-oxo-1-{[2-(pyiridin-4-yl)ethyl]amino}propan-2-yl)phenyl trifluoromethanesulfonate
*N*-(1-ethyl-3-methyl-1*H*-pyrazol-4-yl)-2-[5-(phenylcarbonyl)thiophen-2-yl]propanamide
2-{4-[{3-methoxyphenyl)amino]phenyl}-*N-*(1-benzylpiperidin-4-yl)propanamide
2-[(3-methoxyphenyl)amino]phenyl}-*N*-(1,3-dimcthyl-1*H*-pyrazol-5-yl) propanamide
*N*-(1,3-dimethyl-1*H*-pyrazol-5-yl)-2-[3-(3-fluorophenoxy)phenyl] propanamide
2-[3-(3-fluorophenoxy)phenyl]-*N*-[2-(phenylamino)ethyl]propanamide
2-{4-[(2,6-dichlorophenyl)amino]phenyl}-*N*-phenylpropanamide
2-[3-(cyclopropylamino)phenyl]-N-(pyrimidin-4-yl)propanamide
2-(3-{[4-(morpholin-4-yl)phenyl]amino}phenyl)N-(pyrimidin-4-yl)propanamide
2-{4-[(2,6-dichloro-3-methylphenyl)amino]phenyl}-*N*-[2-(morpholin-4-yl)ethyl]propanamide
2-{4-[(2,6-dichloro-3-methylphenyl)amino]phenyl}-*N*-[2-(cyclohexylamino)propyl] propanamide
*N*-(2-amino-2-methylpropyl)-2-{3-[3-(trifluoromethoxy)phenoxy] phenylpropanamide
*N-*[(2-pyrrolidin-1-yl)ethyl]-2-{3-[3-(trifluoromethoxy)phenoxy]phenyl}propanamide 3-(1-{[2-(4-fluorophenoxy)ethyl]amino}-1-oxopropan-2-yl)phenyl trifluoromethanesulfonate
2-{4-[(propan-2-ylsulfonyl)amino]phenyl}-*N*-(4-tert-butyl-1,3-thiazol-2-yl) propanamide *N*-{2-[(3-methoxybenzyl)(metliyl)amino]ethyl}-2-(4-[(propan-2-ylsulfonyl)amino] phenylpropanamide
*N*-(2-methylprop-2-en-1-yl)-2-[3-(thiophen-2-ylcarbonyl)phenyl] propanamide
*N*-(1,3-dimethyl-1*H*-pyrazol-5-yl)-2-[3-(thiophen-2-ylcarbonyl)phenyl] propanamide 2-{4-[(2,3-dimethoxyphenyl)amino]phenyl}-*N*-(1,3-dimethyl-1*H*-pyrazol-5-yl) propanamide
2-{4-[(2,3-dimethoxyphenyl)amino]phenyl}-*N*-(pyrimidin-4-yl)propanamide
2-{3-[hex-1-en-1-yl]phenyl}-*N*-[2-(propan-2-ylamino)ethyl]propanamide
2-{3-[hex-1-en-1-yl]phenyl}-*N*-(pyrimidin-4-yl)propanamide
*N*-(3-ethyl-1*H*-pyrazol-5-yl)-2-(4-{[4-(trifluoromethyl)-1,3-oxazol-2-yl]amino}phenyl) propanamide
*N*-[2-(*tert*-butylamino)-2-oxoethyl]-2-(4-{[4-(trifluoromethyl)-1,3-oxazol-2-yl]amino} phenyl)propanamide
*N*-{2-[(3-methoxybenzyl)(methyl)amino]ethyl}-2-(4-{[4-(trifluoromethyl)-1,3-oxazol-2-yl]amino}phenyl)propanamide
*N*-[2-hydroxy-3-(3,4-dimethylphenoxy)propyl]-2-(4-{[4-(trifluoromethyl)-1,3-oxazol-2-yl]amino}phenyl)propanamide
2-[3-(phenylcarbonyl)phenyl]-*N*-(1,3-thiazol-2-yl)propanamide
*N-*cyclohexyl-2-[3-(phenylcarbonyl)phenyl]propanamide
*N*-phenyl-2-[3-(phenylcarbonyl)phenyl]propanamide
*N*-(1,3-dimethyl-1*H*-pyrazol-5-yl)-2-[3-(phenylcarbonyl)phenyl] propanamide
2-[4-(2-methylpropyl)phenyl]-*N*-(pyridin-4-yl)propanamide
*N*-carbamoyl-2-[4-(2-methylpropyl)phenyl]propanamide
1-methyl-4-({2-[4-(2-methylpropyl)phenyl]propanoyl}amino)pyrimidin-1-ium iodide
*N*-(1,3-dimethyl-1*H*-pyrazol-5-yl)-2-[4-(2-methylpropyl)phenyl] propanamide
*N*-(1-ethyl-3-methyl-1*H*-pyrazol-5-yl)-2-(4-{[4-(trifluoromethyl)-1,3-thiazol-2-yl]amino}phenyl)propanamide
*N*-[2-(3,5-dimethylpiperidin-1-yl)ethyl]-2-(4-{[4-(trifluoromethyl)-1,3-thiazol-2-yl]amino}phenyl)propanamide
*N*-[furan-2-yl(morpholin-4-yl)methyl]-2-(4-{[4-(trifluoromethyl)-1,3-thiazol-2-yl]amino} phenyl)propanamide
*N*-[4-(pyridin-4-ylmethyl)phenyl]-2-(4-{[4-(trifluoromethyl)-1,3-thiazol-2-yl]amino} phenyl) propanamide
*N*-[2-(furan-2-yl)propyl]-2-(4-{[4-(trifluoromethyl)-1,3-thiazol-2-yl]amino}phenyl) propanamide
4-(1-{[2-(furan-2-yl)propyl]amino}-1-oxopropan-2-yl)phenyl trifluoromethanesulfonate
4-[1-oxo-1-(pyridin-4-ylamino)propan-2-yl]phenyl trifluoromethanesulfonate
4-{1-oxo-1-[4-(pyridin-4-ylmethyl)propan-2-yl]amino}phenyl trifluoromethanesulfonate
4-(1-{[(dimethylamino)(4-fluorophenyl)methyl]amino}-1-oxopropan-2-yl)phenyl trifluoromethanesulfonate
4-(1-{[3-[3-methoxybenzyl(methyl)amino]propyl]amino-1-oxopropan-2-yl)phenyl trifluoromethanesulfonate
4-[3-(3,4-dimethylphenoxy)-2-hydroxypropyl]amino-1-oxopropan-2-yl)phenyl trifluoromethanesulfonate
2-(3-{[3-methoxy-5-(trifluoromethyl)phenyl]amino}phenyl)-*N*-(3-ethoxypropyl) propanamide
2-(3-{[3-methoxy-5-(trifluoromethyl)phenyl]amino}phenyl)-*N*-(1*H*-pyrrol-1-yl) propanamide
*N*'-{2-[3-(3-methoxy-5-(trifluoromethyl)phenylammo)phenyl]propanoyl} furan-2-carbohydrazide
2-(3-{[3-methoxy-5-(trifluoromethyl)phenyl]amino}phenyl)-*N*-(pyrimidin-4-yl) propanamide
*N*-ethyl-2-(3-{[3-methoxy-5-(trifluoromethyl)phenyl]amino}phenyl)propanamide
2-{3-[(3-methoxy-5-(trifluoromethyl}phenyl}amino]phenyl}-*N-*[2-(benzylamino)ethyl] propanamide
*N*-(2-amino-2-methylpropyl)-2-[3-{[3-methoxy-5-(trifluoromethyl)phenyl]amino}phenyl] propanamide
*N*-(2-aminocyclohexyl)-2-[3-{[3-methoxy-5-(trifluoromethyl)phenyl]amino}phenyl] propanamide
Methyl 3-[(tert-butoxycarbonyl)amino]-2-[4-(naphthalen-1-yloxyphenyl)propanoyl] aminopropanoate
*N*-[2-(benzylamino)ethyl]-2-[4-(naphthalen-1-yloxy)phenyl]propanamide
*N*-[3-(dimethylamino)propyl]-2-[4-(naphthalen-1-yloxy)phenyl]propanamide
*N*-[3-(cyclohexylamino)propyl]-2-[4-(naphthalen-1-yloxy)phenyl]propanamide
2-[4-(naphthalen-1-yloxy)phenyl]-*N*-(4*H*-1,2,4-triazol-4-yl)propanamide
2-[4-(naphthalen-1-yloxy)phenyl]-*N*-[2-(1-methylpyrrolidin-2-yl)ethyl] propanamide
*N*-[2-(acetylamino)ethyl]-2-[4-(naphthalen-1-yloxy)phenyl]propanamide
2-[4-(naphthalen-1-yloxy}phenyl]-*N-*[2-(morpholin-4-yl)ethyl] propanamide

The above 2-arylpropionamide derivatives show the ability to effectively inhibit bradykinin biological activity due to their nature of selective BKB1R antagonists.

Thus, a further object of the present invention are the above compounds for use as inhibitors of bradykinin B1 receptor-activated pathway.

As it has already been discussed in the background of the invention this pathway is responsible for the pathogenesis of disorders involving pain and inflammation.

Accordingly, a further object of the present invention is the use of the above compounds for prevention and/or treatment of pain and inflammation.

In particular, object of the present invention is the use of the above compounds for the prevention and/or treatment of visceral pain, preferably pancreatitis, cystitis, renal colic; neuropathic pain, preferably post herpetic neuralgia, nerve injury; central pain syndromes caused by virtually any lesion at any level of the nervous system and postsurgical pain syndromes; bone and joint pain, preferably osteoarthritis; repetitive motion pain; dental pain; cancer pain; myofascial pain, preferably muscular injury, fibromyalgia; perioperative pain; chronic pain; dysmenorrea; pain associated with angina and inflammatory pain of varied origins, preferably osteoarthritis, rheumatoid arthritis, rheumatic disease and gout.

A further object of the invention is the use of the compounds of the invention for the prevention and/or treatment of hyperreactive airways and inflammatory events associated with airway disease, preferably asthma including allergic asthma, bronchoconstriction, occupational asthma, viral- or bacterial-exacerbation of asthma, other non-allergic asthmas and "wheezy-infant syndrome", chronic obstructive pulmonary disease. Preferably said chronic obstructive pulmonary disease comprises emphysema, ARDS, bronchitis, pneumonia, allergic and vasomotor rhinitis.

### Preferably, said pneumoconiosis comprises aluminosis, anthracosis, asbestosis, chalicosis, ptilosis, siderosis, tabacosis and byssinosis

A still further object of the present invention is the use of the above compounds for prevention and/or treatment of inflammatory bowel diseases, prefarbly Crohn's disease and ulcerative colitis and uveitis; inflammatory skin disorders, prefrably psoriasis and eczema; edema resulting from burns, sprains and fractures; cerebral edema and angioedema; diabetic vasculopathy; diabetic neuropathy; diabetic retinopathy; diabetic symptoms associated with insulitis; liver disease; multiple sclerosis; cardiovascular disease, preferably atherosclerosis; congestive heart failure; myocardial infarct; neurodegenerative diseases, preferably Parkinson's and Alzheimer's disease; epilepsy; septic shock; headache including cluster headache, migraine including prophylactic and acute use; closed head trauma; cancer; sepsis; gingivitis; osteoporosis; benign hyperplasia and hyperactive bladder.

Pharmaceutical compositions comprising a compound of the invention and a suitable carrier thereof, are also within the scope of the present invention.

The compounds of the invention, together with a conventionally employed adjuvant, carrier, diluent or excipient may, in fact, be placed into the form of pharmaceutical compositions and unit dosages thereof, and in such form may be employed as solids, such as tablets or filled capsules, or liquids such as solutions, suspensions, emulsions, elixirs, or capsules filled with the same, all for oral use, or in the form of sterile injectable solutions for parenteral (including subcutaneous) use. Such pharmaceutical compositions and unit dosage forms thereof may comprise ingredients in conventional proportions, with or without additional active compounds or principles, and such unit dosage forms may contain any suitable effective amount of the active ingredient commensurate with the intended daily dosage range to be employed.

When employed as pharmaceuticals, the acids of this invention are typically administered in the form of a pharmaceutical composition. Such compositions can be prepared in a manner well known in the pharmaceutical art and comprise at least one active compound.

Generally, the compounds of this invention are administered in a pharmaceutically effective amount. The amount of the compound actually administered will typically be determined by a physician, in the light of the relevant circumstances, including the condition to be treated, the chosen route of administration, the actual compound administered, the age, weight, and response of the individual patient, the severity of the patient's symptoms, and the like.

The pharmaceutical compositions of the invention can be administered by a variety of routes including oral, rectal, transdermal, subcutaneous, intravenous, intramuscular, and intranasal. Depending on the intended route of delivery, the compounds are preferably formulated as either injectable or oral compositions. The compositions for oral administration can take the form of bulk liquid solutions or suspensions, or bulk powders.

More commonly, however, the compositions are presented in unit dosage forms to facilitate accurate dosing. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient. Typical unit dosage forms include prefilled, premeasured ampoules or syringes of the liquid compositions or pills, tablets, capsules or the like in the case of solid compositions. In such compositions, the acid compound is usually a minor component (from about 0.1 to about 50% by weight or preferably from about 1 to about 40% by weight) with the remainder being various vehicles or carriers and processing aids helpful for forming the desired dosing form.

Liquid forms suitable for oral administration may include a suitable aqueous or nonaqueous vehicle with buffers, suspending and dispensing agents, colorants, flavors and the like. Liquid forms, including the injectable compositions described herebelow, are always stored in the absence of light, so as to avoid any catalytic effect of light, such as hydroperoxide or peroxide formation. Solid forms may include, for example, any of the following ingredients, or compounds of a similar nature: a binder such as microcrystalline cellulose, gum tragacanth or gelatine; an excipient such as starch or lactose, a disintegrating agent such as alginic acid, Primogel, or corn starch; a lubricant such as magnesium stearate; a glidant such as colloidal silicon dioxide; a sweetening agent such as sucrose or saccharin; or a flavoring agent such as peppermint, methyl salicylate, or orange flavoring.

Injectable compositions are typically based upon injectable sterile saline or phosphate-buffered saline or other injectable carriers known in the art. As above mentioned, the acid derivative of formula I in such compositions is typically a minor component, frequently ranging between 0.05 to 10% by weight with the remainder being the injectable carrier and the like. The mean daily dosage will depend upon various factors, such as the seriousness of the disease and the conditions of the patient (age, sex and weight). The dose will generally vary from 1 mg or a few mg up to 1500 mg of the compounds of formula (1) per day, optionally divided into multiple administrations. Higher dosages may be administered also thanks to the low toxicity of the compounds of the invention over long periods of time.

The above described components for orally administered or injectable compositions are merely representative. Further materials as well as processing techniques and the like are set out in Part 7 of "Remington's Pharmaceutical Sciences Handbook", 19th Edition, 1995, Mack Publishing Company, Easton, Pennsylvania, which is incorporated herein by reference.

The compounds of the invention can also be administered in sustained release forms or from sustained release drug delivery systems. A description of representative sustained release materials can also be found in the incorporated materials in the Remington's Handbook as above.

The present invention shall be illustrated by means of the following examples which are not construed to be viewed as limiting the scope of the invention.

### Materials and methods

The amines of general formula RNH₂ used as reagents in the synthesis of compounds of formula (I) are known products, generally commercially available, or they can be prepared according to methods described in the literature.

The following arylpropionic acids have been already described or are commercially available: 2-[4-(2-methylpropyl)phenyl]propanoic acid, 2-[3-(phenylcarbonyl)phenyl] propanoic acid, 2-(4-{[(trifluoromethyl)sulfonyl]oxy}phenyl)propanoic acid, 2-{4-[(propan-2-ylsulfonyl)oxy]phenyl}propanoic acid, 2-fluoro-2-(4-{[(trifluoromethyl)sulfonyl]oxy}phenyl)propanoic acid, 2-(4-{[4-(trifluoromethyl)-1,3-thiazol-2-yl]amino}phenyl)propanoic acid, 2-(4-{[4-(trifluoromethyl)-1,3-oxazol-2-yl]amino}phenyl)propanoic acid, 2-[3-(thiophen-2-ylcarbonyl)phenyl]propanoic acid, 2-fluoro-2-(3-{[4-(trifluoromethyl)-1,3-thiazol-2-yl]amino}phenyl)propanoic acid.

¹H-NMR spectra were recorded on a Bruker ARX 300 spectrometer. LC-MS spectra were recorded on a Surveyor (THERMO FINNIGAN) apparatus coupled with a LCQ DECA XP plus (THERMO FINNIGAN) apparatus and equipped with a C18 Phenomenex Gemini column. The eluent mixture consisted of buffer 10 mM pH 4.2 HCOO⁻ NH4⁺/HCOOH and CH₃OH used according the gradient from 90:10 to 10:90.

### Synthesis of arylpropionic acids

### 2-[4-(Naphthalen-1-yloxy)phenyl]propanoic acid (I)

In a 500 cc round-bottomed flask equipped with condenser and magnetic stirrer, at room temperature commercial 2-(4-hydroxyphenyl)propanoic acid (20 g, 0.12 mol) was dissolved in CH₃OH (50ml) and conc. H₂SO₄ (2 mL, 0.05 mol) was added dropwise to the solution. The reaction mixture was left refluxing overnight. After cooling at room temperature, the solution was diluted in CH₂Cl₂ (20 ml) and extracted with a saturated NaHCO₃ aqueous solution (3 x 150ml); the collected organic layers were dried over anhydrous Na₂SO₄ and evaporated under vacuum to give pure methyl 2-(4-hydroxyphenyl)propanoate (18 g, 0.10 mol) as orange oil, used for the next step reaction without further purification.

In a 50 ml round-bottomed flask under nitrogen flux, Cu(OAC)₂ (0.49 g, 2.7 mmol) and a catalytic amount of 4Å molecular sieves were suspended in dry CH₂Cl₂ (0.1 mL). After stirring for 10 min. pyridine (0.45 ml, 5.4 mol) was added. The nitrogen inlet was removed and substituted by a CaCl₂-tube; a solution of methyl-2-(4-hydroxyphenyl)propanoate (0.5 g, 2.7 mmol) in pyridine (0.65 ml, 8.1 mmol) was added, immediately followed by small positions of naphthylboronic acid (0.7 g, 4.5 mmol). The mixture was left stirring at room temperature for 3 days and, at the completion of the reaction, the solvents were evaporated under vacuum. The crude mixture was purified by flash chromatography (petroleum ether/EtOAc 8:1) to afford the intermediate methyl 2-[4-(naphthalene-1-yloxy)phenyl] propanoate (0.55 g, 1.8 mmol) used for the following step without further purification.

To a suspension of methyl-2-[4-(naphtalen-1-yloxy)phenyl]propanoate (0.5 g, 1.63 mmol) in dioxane (2 mL) at room temperature, 2M NaOH (4 mL, 0.8 mmol) was added dropwise. The resulting dark solution was stirred at room temperature for 8 h, then it was evaporated under vacuum, the crude dissolved in EtOAc (3 mL) and extracted with water (3 x 5 mL). The collected aqueous extracts were then acidified with 10% _{w/v} KHSO₄ (2 mL) and back extracted with EtOAc (3 x 5 mL). The collected organic layer was dried over anhydrous Na₂SO₄ and evaporated under vacuum to give a crude that, after pulping in petroleum ether afforded pure 2-[4-naphthalen-1-yloxy)phenyl]propanoic acid (I) (0.3 g, 1.027 mmol, 63 % yield from the last intermediate) as waxy solid. ¹H-NMR (CDCl₃) δ 8.2 (d, 1H., J=7.9 Hz), 7.90 (d, 1H, J=7.6 Hz), 7.65 (d, 1H, J=8.1 Hz), 7.6-7.46 (m, 2H), 7.41 (t, 1H, J=8.1 Hz), 7.32 (d, 2H, J=8.1 Hz), 7.1-6.95 (m, 3H), 3.8 (q, 1H, J=7 Hz), 1.55 (d, 3H, J=7 Hz). MS (ESI): m/z [M+H]¹⁺ 292 .

### 2-(3-{[3-Methoxy-5-(trifluoromethyl)phenyl]amino}phenyl)propanoic acid (II)

In a 250 ml round-bottomed flask equipped with condenser, few drops of conc. H₂SO₄ were added to a solution of commercial (3-hydroxyphenyl)acetic acid (25 g, 0.165 mol) in CH₃OH (30 mL), and the resulting mixture was left stirring under reflux for 4 h. After complete disappearance of the starting material (TLC) CHCl₃ (30 mL) was added and the organic layer was extracted with 1M NaOH (2 x 20mL), washed with water (2 x 20 mL), dried over anhydrous Na₂SO₄ and evaporated under vacuum to give pure methyl(3-hydroxyphenyl)acetate (24 g, 0.14 mol) as a brown oil which was used for the next step without any further purification.

In a three necks 250 ml round-bottomed flask equipped with dropping funnel and magnetic stirrer, a solution of methyl(3-hydroxyphenyl)acetate (10g, 0.058 mol) in CH₃OH (50 mL) was cooled to -20°C and triethylamine (12.4 mL, 0.075 mol) was added dropwise; the resulting mixture was left stirring for 30 min. and then trifluoromethanesulfonic anhydride (12.6 mL, 0,075 mol) was added, the ice-water bath removed and the solution stirred for further 2 h. After complete disappearance of the starting material (TLC) the reaction mixture was diluted with IN HCl (30 mL) and washed with water (2 x 10 mL), dried over anhydrous Na₂SO₄ and evaporated under vacuum to give methyl (3-{[(trifluoromethyl)sulfonyl]oxy}phenyl)acetate (9 g, 0.031 mol) as yellow oil used for the next step without any further purification.

In a 250 ml round-bottomed flask lithium hexamethyldisilazide (1M in THF, 31.4 mL, 0.031 mol) and methyl(3-{[trifluoromethyl)sulfonyl]oxy}phenyl)acetate (8.9 g, 0.03 mol) were dissolved in anhydrous THF (170 ml) under nitrogen atmosphere. After cooling to -78°C and stirring for 20 min, iodomethane (1.86 mL, 0.03 mol) was added dropwise. The resulting mixture was left warming up to room temperature and stirred overnight. An aqueous solution of KH₂PO₄ (30 ml) was added and the aqueous layer extracted with EtOAc (2 x 20 mL). The collected organic extracts were washed with water (2 x 20 ml), dried over anhydrous Na₂SO₄ and evaporated under reduced pressure to give a crude residue that, after purification by flash chromatography (n-hexane/EtOAc 9:1), afforded methyl 2-(3-{[(trifluoromethyl)sulfonyl]oxy}phenyl)propanoate (7.5 g, 0.025 mol, 75% yield) as yellow oil used for the next step.

To a solution of methyl 2-(3-{[(trifluoromethyl)sulfonyl]oxy}phenyl)propanoate (0.13 g, 4.3 mmol) and 3-methoxy-5-(trifluoromethyl)aniline (0.1 g, 5.2 mmol) in dry toluene (2 mL), Pd₂(dba)₃ (8 mg, 8.7 mmol), Xantphos (7.5 mg, 1.3 mmol) and K₃PO₄ (0.18 g, 8.7 mmol) were added. The reaction mixture was heated at 150°C and stirred under microwave irradiations. After stirring for 2 h the solvent was evaporated and the residue purified by flash chromatography (n-hexane/EtOAc 9:1) to afford methyl 2-(3-{[3-methoxy-5-(trifluoromethyl)phenyl]amino}phenyl)propanoate (0.8 g, 2.4 mmol, yield 56 %) pure enough for the next step.

In a 100ml round-bottomed flask equipped with condenser and magnetic stirrer, methyl 2-(3-{[3-methoxy-5-(trifluoromethyl)phenyl]amino}propanoate (0.73 g, 2.1 mmol) was suspended in 1,4-dioxane (20 mL) at room temperature, 1M NaOH (4.2 mL, 4.2 mmol) was added by dripping and the resulting solution was stirred at room temperature overnight. The solvents were evaporated under vacuum and the crude mixture diluted in EtOAc (10 mL) and extracted with water (3 x 10 mL). The collected aqueous layers were acidified to pH 2 with IN HCl, washed with brine (2 x 5 mL) and extracted with EtOAc (3 x 10 mL). The organic layer was dried anhydrous Na₂SO₄ and evaporated under reduced pressure to give 2-(3-{[3-methoxy-5-(trifluorornethyl)phenyl]amino}-phenyl)propanoic acid (II) (0,72 g, 2.0 mmol, yield 95%) as yellow oil. ¹H-NMR (CD₃OD): δ 7.9 (bs, 1H, NH), 7.2 (t, 1H, J=8 Hz), 7.12 (s, 1H), 7.0 (dd, 1H, J¹=7.8 Hz, J²=1, Hz), 6.90 (d, 1 H, J=7,8Hz), 6.83 (s, 1H), 6.80 (s, 1H), 6.56 (s, 1H), 3.77 (s, 3H), 3.65 (q, 1H, J=7Hz), 1.43 (d, 3H, J=7Hz). MS (ESI): m/z [M+H]¹⁺ 340.

### 2-(3-{[(Trifluoromethyl)sulphonyl]oxy}phenyl)propanoic acid (III)

In a 500 cc round-bottomed flask equipped with condenser and magnetic stirrer, methyl 2-(3-{[(trifluoromethyl)sulfonyl]oxy}phenyl)propanoate (1.2 g, 3.8 mmol) (prepared as above described) was suspended in acetic acid (10 mL) at room temperature and 37% HCl (5 mL) was added by dripping. The resulting solution was refluxed overnight. After cooling at room temperature, the solution was evaporated under vacuum and the crude mixture diluted in CH₂Cl₂ (10 mL) and washed with water (2 x 10 mL); the organic layer was dried over anhydrous Na₂SO₄ and evaporated under reduced pressure to give pure 2-(3-{[trifluoromethyl)sulfony]oxy}phenyl)propanoic acid (III) (1.09 g, 3.6 mmol, yield 95%) as colourless oil. ¹H-NMR (CDCl₃): δ 7.50-7.35 (m, 2H), 7.27 (s, 1H), 7.22 (d, 1H, J=7.3Hz), 3.83 (q, 1H, J=7.3Hz), 1.58 (d, 3H, J=7.3Hz). MS (ESI): m/z [M+H]¹⁺ 299.

### 2-{4-[(2,3-Dimethoxyphenyl)amino]phenyl}propanoic acid (IV)

Following the same above described procedure for the synthesis of II, but starting from commercial (4-hydroxyphenyl)acetic acid, the intermediate ethyl 2-(4-{[(trifluoromethyl)sulfonyl]oxy}phenyl)propanoate was isolated pure as brown oil and, after treatment with commercial 2,3-dimethoxyaniline in the same conditions previously described and following ester hydrolysis, afforded 2-{4-[(2,3-dimethoxyphenyl)amino]phenyl}propanoic acid (IV) in 25% overall yield as a colourless oil. ¹H-NMR (CDCl₃): δ 9.25 (bs, 1H), 7.60 (t, 1H, J=7Hz), 7.41 (d, 2H, J=8.9Hz), 7.32-7,20 (m, 4H), 3.8 (q, 1H, J=7.3Hz), 1.59 (d, 3H, J=7,3Hz). MS (ESI): m/z [M+H]¹⁺ 302.

### 2-13-[3-(Trifluoromethoxy)phenoxy]phenyl}propanoic acid (V)

Commercial (3-hydroxyphenyl)acetic acid was methylated by iodomethane following the procedure above described for I to give the intermediate methyl 2-(3-hydroxyphenyl)propanoate that, following treatment with commercial 3-(trifluoromethoxy)phenylboronic acid in the same conditions previously described for compound (I) afforded 2-{3-[3-(trifluoromethoxy)phenoxy]pheny}propanoic acid (V) in 23% overall yield as a colourless oil. ¹H-NMR (CDCl₃₎:□ 7.60-7.35 (m, 5H), 7.27 (s, 1H), 7.20-7.0 (m, 2H), 3.83 (q, 1H, J=7.3Hz), 1.58 (d, 3H, J=7.3Hz). MS (ESI): m/z [M+H]¹⁺ 327.

### 2-{4-[2,6-Dichloro-3-methylphenyl)amino]phenyl}propanoic acid (VI)

Following the same above described procedure for the synthesis of II, but starting from commercial (4-hydroxyphenyl)acetic acid, the intermediate methyl 2-(4-{[(trifluoromethyl)sulfonyl]oxy}phenyl)propanoate was isolated pure as brown oil and, after treatment with commercial 2,6-dichloro-3-methylaniline in the same conditions previously described and following ester hydrolysis, afforded 2-{4-[2,6-dichloro-3-methylphenyl)amino]phenyl}propanoic acid (VI) in 28% overall yield as a colourless oil. ¹H-NMR (CDCl₃): δ 8.7 (bs, 1H), 7.41 (d, 2H, J=8.9Hz), 7.20 (d, 2H, J=8.9Hz), 7.02 (d, 1H, J=7.2Hz), 6.80 (d, 1H, J=7.2Hz), 3.8 (q, 1H, J=7.3Hz), 2.3 (s, 3H), 1.59 (d, 3H, J=7.3Hz). MS (ESI): m/z [M+H]¹⁺ 325.

### 2-(4-{[4-(Morpholin-4-yl)phenyl]amino}phenyl)propanoic acid (VII)

Following the same above described procedure for the synthesis of II, but starting from commercial (4-hydroxyphenyl)acetic acid, the intermediate-methyl-2-(4-{[(trifluoromethyl)-sulfonyl]oxylphenyl)-propanoate was isolated pure as brown oil and, after treatment with commercial 4-morpholinoaniline in the same conditions previously described and following ester hydrolysis, afforded 2-(4-{[4-(morpholin-4-yl)phenyl]amino}phenyl)propanoic acid (VII) in 34% overall yield as a colourless oil. ¹H-NMR (CDCl₃): δ 8.5 (bs, 1H), 7.41 (d, 2H, J=8.9Hz), 7.20 (d, 2H, J=8.9Hz), 7.0 (d, 2H, J=7.2Hz), 6.7 (d, 2H, J=7.2Hz), 3.8 (m, 5H), 3.0 (m, 4H), 1.62 (d, 3H, J=7.3Hz). MS (ESI): m/z [M+H]¹⁺ 327.

### 2-{4-[(2,6-Dichlorophenyl)amino]phenyl}propanoic acid (VIII)

Following the same procedure described for the synthesis of VI, but reacting the intermediate methyl 2-(4-{[(trifluoromethyl)sulfonyl]oxy}phenyl)propanoate with commercial 2,6-dichloroaniline, 2-{4-[(2,6-dichlorophenyl)amino]phenyl}propanoic acid (VIII) was isolated as a colourless oil in 30% overall yield. ¹H-NMR (CDCl₃): □ 8.65 (bs, 1H), 7.41 (d, 2H, J=8.9Hz), 7.20 (d, 2H, J=8.9Hz), 7.1 (d, 2H, J=7Hz), 6.90 (t, 1H, J=7Hz), 3.8 (q, 1H, J=7.3Hz), 1.59 (d, 3H, J=7.3Hz). MS (ESI): m/z [M+H]¹⁺ 311.

### 2-{4-[(Cyclopropylmethyl)amino]phenyl}propanoic acid (IX)

Following the same procedure described for the synthesis of VI, but reacting the intermediate methyl 2-(4-{[(trifluoromethyl)sulfonyl]oxy}phenyl)propanoate with commercial cyclopropanemethylamine, 2-{4-[(cyclopropylmethyl)amino]phenyl} propanoic acid (IX) was obtained as a colourless oil in 24% overall yield. ¹H-NMR (CDCl₃): δ 8.65 (bs, 1H), 7.41 (d, 2H, J=8.9Hz), 7.20 (d, 2H, J=8.9Hz), 3.8 (q, 1H, J=7.3Hz), 2.5 (d, 2H, J=7Hz), 1.59 (d, 3H, J=7.3Hz), 0.9 (m, 1H), 0.4 (m, 2H), 0.1 (m, 1H). MS (ESI): m/z [M+H]¹⁺ 220.

### 2-[3-(3-Fluorophenoxy)phenyl]propanoic acid (X)

Following the same procedure described for the synthesis of I, but reacting the intermediate methyl-2-(3-hydroxyphenyl)propanoate with commercial 3-fluorophenylboronic acid, 2-[3-(3-fluorophenoxy)phenyl]propanoic acid (X) was obtained as a colourless oil in 40% overall yield. ¹H-NMR (CDCl₃); δ 7.70-7.40 (m, 5H), 7.27 (s, 1H), 7.20-7.00 (m, 2H), 3.83 (q, 1H, J=7.3Hz), 1.58 (d, 3H, J=7.3Hz). MS (ESI): m/z [M+H]¹⁺ 261.

### 2-{4-[(3-Methoxyphenyl)amino]phenyl}propanoic acid (XI)

Following the same procedure described for the synthesis of VI, but reacting the intermediate methyl 2-(4-{[(trifluoromethyl)sulfonyl]oxy}phenyl)propanoate with commercial 3-methoxyaniline, 2-{4-[(3-methoxyphenyl}amino]phenyl}propanoic acid (XI) was obtained as a colourless oil in 25% overall yield. ¹H-NMR (CDCl₃): δ 8.68 (bs, 1H), 7.41 (d, 2H, J=8.9Hz), 7.20 (d, 2H, J=8.9Hz),7,05 (t, 1H, J=6,8Hz), 6.8-6.6 (m, 3H), 3.8 (q, 1H, J=7.3Hz), 3.70 (s, 3H), 1.59 (d, 3H, J=7.3Hz). MS (ESI): m/z [M+H]¹⁺ 272.

### 2-{4-[3-(Trifluoromethoxy)phenoxy]phenyl}propanoic acid (XII)

Following the same procedure described for the synthesis of I, but reacting the intermediate methyl-2-(4-hydroxyphenyl)propanoate with commercial 3-(trifluoromethoxy)phenylboronic acid, 2-{4-[3-(trifluoromethoxy)phenoxy]phenyl} propanoic acid (XII) was obtained as a colourless oil in 32% overall yield. ¹H-NMR (CDCl₃): δ7.6-7.40 (m, 3H), 7.35-7.30 (m, 3H), 7.0 (d, 2H, J=8.0Hz), 3.8 (q, 1H, J=7Hz), 1.55 (d, 3H, J=7 Hz). MS (ESI): m/z [M+H]¹⁺327.

### 2-Methyl-2-(4-{[(trifluoromethyl)sulfonyl]oxy}phenyl)propanoic acid (XIII)

In a 250 ml round-bottomed flask lithium hexamethyldisilazide (1M in THF, 5 mL, 6.3 mmol) and methyl2-(4-{[trifluoromethyl)sulfonyl]oxy}phenyl)propanoate (1.4 g, 4.5 mmol) were dissolved in anhydrous THF (5 mL) under nitrogen atmosphere. After cooling to -78°C and stirring for 20 min, iodomethane (0.4 mL, 6.3 mmol) was added by dripping. The resulting mixture was left warming up to room temperature and stirred overnight. An aqueous solution of KH₂PO₄ (30 mL) was added and the aqueous layer extracted with EtOAc (2 x 20 mL). The collected organic extracts were washed with water (2 x 20 mL), dried over anhydrous Na₂SO₄ and evaporated under reduced pressure to give a crude residue that, after purification by flash chromatography (n-hexane/EtOAc 9:1), afforded methyl-2-methyl-2-(4-{[(trifluoromethyl)sulfonyl]oxy}phenyl)propanoate (1.2 g, 4.1 mmol, 93% yield) as colourless oil used for the next step.

In a 500 cc round-bottomed flask equipped with condenser and magnetic stirrer, 37% HCl (5mL) was added by dripping at room temperature to a suspension of methyl-2-methyl-2-(4-{[(trifluoromethyl)sulfonyl]oxy}phenyl)propanoate (1.2 g, 4.1 mmol) in AcOH (10 mL). The resulting solution was refluxed under stirring overnight. After cooling at room temperature, solvent was evaporated under vacuum and the crude mixture diluted in CH₂Cl₂ (10mL) and the organic phase was washed with water (2 x 10 mL), dried over anhydrous Na₂SO₄ to give 2-methyl-2-(4-{[(trifluoromethyl)sulfonyl]oxy} phenyl)propanoic acid (XIII) (1.09 g, 3.5 mmol) as colourless oil in 85% yield. ¹H-NMR (CDCl₃): δ 7.40 (d, 2H, J=8.5Hz), 7.24 (d, 2H, J=8.5Hz), 1.63 (s, 6H). MS (ESI): m/z [M+H]¹⁺313,

### 2-{3-[(1-hex-1-en-1-yl]phenyl}propanoic acid (XIV)

To a solution of methyl 2-(3-{[(trifluoromethyl)sulfonyl]oxy}phenyl)propanoate (prepared as above described for II) (0.52 g, 2.24 mmol) in anhydrous N-methyl-2-pyrrolidinone (6 mL) under nitrogen and vigorous stirring, LiCl (0.285 g, 6.73 mmol), CuI (20 mg, 0.112 mmol), AsPh₃ (54 mg, 0.18 mmol), and Pd₂dba₃ (42 mg, 0.04 mmol) were added. After stirring for 10 min, 1-hexenyl-tributyltin (prepared according Labadie J.W. et al. J. Org. Chem., 1983, 105, 6129-6137) (1.01 g, 2.70 mmol) was added and the reaction mixture left stirring for 5 h at 90°C. After cooling at room temperature, a saturated solution of KF (20 mL) was added and the aqueous layer extracted with Et₂O (3 x 20 mL). The collected organic extracts were washed with water (2 x 20 mL), dried over anhydrous Na₂SO₄ and evaporated under vacuum to give a crude residue that, after purification by flash chromatography (petroleum ether/ EtOAc 8:1), afforded methyl 2-{3-[(1-hex-1-en-1-yl]phenyl}propanoate (0.41g, 1.68 mmol, 75% yield) used for the final step of hydrolysis.

To the methyl ester was dissolved in CH₃OH (4 mL) a 20% alcoholic solution of KOH (2 mL) was added, and the resulting mixture was left stirring overnight at room temperature.

After solvent evaporation the residue was diluted with water (10 mL) and washed with Et₂O (2 x 10 mL), acidified with IN HCl to pH 2 and extracted with EtOAc (2 x 20 mL). The collected organic extracts were dried over anhydrous Na₂SO₄ and evaporated under vacuum to give a crude residue that, after pulping in petroleum ether (20 mL) and filtration, afforded 2-{3-[(hex-1-en-1-yl]phenyl}prapanoic acid (XIV) (0.35 g, 1.51 mmol, 90% yield) as white solid as a 3:1 mixture of E/Z isomers, ¹H-NMR (CDCl₃): □ 7.60-7.45 (m, 2H), 7.35 (s, 1H), 7.22 (d, 1H, J=7.3Hz), 6.50-6.10 (m, 2H), 3.83 (q, 1H, J=7.3Hz), 2.00 (m, 2H), 1.58 (d, 3H, J=7.3Hz), 1.50-1.15 (m, 4H), 0.9 (t, 3H, J=6.9Hz). MS (ESI) : m/z [M+H]¹⁺ 233.

### General synthesis of amides of formula (I)

### Example 1

### 4-(1-amino-2-fluoro-1-oxopropan-2-yl)phenyl trifluoromethanesulfonate

2-metltyl-2-(4-{[(trifluoromethyl}sulfonyl]oxy}phenyl)propanoic acid (0.88 g, 2.78 mmol) was dissolved in SOCl₂ (5 mL) and the resulting solution was left stirring at reflux 3h. After cooling at room temperature, the mixture was evaporated under reduced pressure; the crude acyl chloride was diluted with anhydrous THF (5 mL) and cooled at 0-5°C. Gaseous ammonia was bubbled into the solution up to the complete disappearance of the acid (the reaction was monitored by TLC). The solvent was evaporated under reduced pressure and the residue diluted with CH₂Cl₂ (10 mL) and washed with a saturated solution of NaHCO₃ (3 x 10 mL) and H₂O (2 x 10 mL); the organic layer was dried over anhydrous Na₂SO₄ and evaporated under vacuum and the crude pulped in isopropyl ether to give, after filtration, the pure 4-(1-amino-2-fluoro-1-oxopropan-2-yl)phenyl trifluoromethanesulfonate (0.74 g, 85% yield) as white solid. ¹H-NMR (CDCl₃) δ 7.75 (d, 2H, J = 7Hz), 7.30 (d, 2H, J = 7Hz), 6.42 (bs, 1H, CONH), 5.47 (bs, 1H, CONH), 1.95 (d, 3H, J = 23Hz).

### Example 2

### 4-(2-fluoro-1-{[2-(5-methyl-1H-pyrazol-1-yl)ethyl]amino}-1-oxopropan-2-yl)phenyl trifluoromethanesulfonate

2-methyl-2-(4-{[(trifluoromethyl)sulfonyl]oxy}phenyl)propanoic acid (0.88 g, 2.78 mmol) was dissolved in SOCl₂ (5 mL) and the resulting solution was left stirring at reflux 3h. After cooling at room temperature, the mixture was evaporated under reduced pressure; the crude acyl chloride was diluted with dry THF (5 mL) and cooled at 0-5°C. 2-(5-methyl-1H-pyrazol-1-yl)ethanamine (0.76 g, 6.11 mmol) (prepared as described in Attaryan O.S. et al., Russ. J. Gen. Chem., 2008, 78, 136-138) was added to the mixture, under vigorous stirring.

The reaction was monitored by TLC and left stirring at room temperature for 2-4 h; after the complete disappearance of the starting acid the solvent was evaporated under reduced pressure and the residue diluted with CH₂Cl₂ (10 mL) and H₂O (10 mL); the two phases were debated and separated and the organic one was washed with a saturated solution of NaHCO₃ (3 x 10 mL) and H₂O (2 x 10 mL), dried over Na₂SO₄ and evaporated under vacuum to give pure 4-(2-fluoro-1-{[2-(5-methyl-1*H*-pyrazol-1-yl)ethyl]amino}-1-oxopropan-2-yl)phenyl trifluoromethanesulfonate (0.82 g, 70% yield) as colourless oil.
MS (ESI) : m/z [M+H]¹⁺ 424 Rt = 1.65 min.

According to the same experimental procedure and using the corresponding above described arylpropionic acids as starting reagents, the following compounds were synthesized:

### Example 3

### 4-(2-fluoro-1-oxo-1-{[2-(pyridin-2-ylamino)ethyl]amino}propan-2-yl)phenyl trifluoromethanesulfonate

MS (ESI) : m/z [M+H]¹⁺ 436; Rt = 1.67 min,

### Example 4

### 2-fluoro-N-(2-sulfamoylthiophen-3-yl)-2-(3-{[4-(trifluoromethyl)-1,3-thiazol-2-yl)amino}phenyl)propanamide

MS (ESI) : m/z [M+H]¹⁺ 495; Rt = 3.86 min.

### Example 5

### 2-fluoro-N-(2-sulfamoylphenyl)-2-(3-{[4-(trifluoromethyl)-1,3-thiazol-2-yl]amino} phenyl)propanamide

MS (ESI) : m/z [M+H]¹⁺ 489; Rt = 3.88 min.

### Example 6

### 4-(2-methyl-1-{[2-(tert-butylamino)-2-oxoethy]amino}-1-oxopropan-2-yl)phenyl trifluoromethanesulfonate

MS (ESI) : m/z [M+H]¹⁺ 425; Rt = 1.67 min.

### Example 7

### 4-(2-methyl-1-oxo-1-{[2-(pyridin-4-yl)ethyl]amino}propan-2-yl)phenyl trifluoromethanesulfonate

MS (ESI) : m/z [M+H]¹⁺ 417; Rt = 1.64 min.

### Example 8

### N-(1-ethyl-3-methyl-1H-pyrazol-4-yl)-2-[5-(phenylcarbonyl)thiophen-2-yl]propanamide

MS (ESI) : m/z [M+H]¹⁺ 368; Rt = 1.22 min.

### Example 9

### 2-{4-[(3-methoxyphenyl)amino]phenyl}-N-(1-benzylpiperidin-4-yl)propanamide

MS (ESI) : m/z [M+H]¹⁺ 444; Rt = 9.72 min.

### Example 10

### 2-[(3-methoxyphenyl)amino]phenyl}-N-(1,3-dymethyl-1H-pyrazol-5-yl)propanamide;

MS (ESI) : m/z [M+HCOOH-H]¹⁻409; Rt = 10.55 min.

### Example 11

### N-(1,3-dimethyl-1H-pyrazol-5-yl)-2-[3-(3-fluorophenoxy)phenyl]propanamide;

MS (ESI): m/z [M+H]¹⁺ 353; Rt = 11.34 min.

### Example 12

### 2-[3-(3-fluorophenoxy)phenyl]-N-[2-(phenylamino)ethyl]propanamide

MS (ESI) : m/z [M+H]¹⁺ 393; Rt = 9.50 min,

### Example 13

### 2-{4-[(2,6-dichlorophenyl)amino]phenyl}-N-phenylpropanamide

MS (ESI) : m/z [M+H]¹⁺ 439; Rt = 9.84 min.

### Example 14

### 2-[3-(cyclopropylamino)phenyl]-N-(pyrimidin-4-yl)propanamide

MS (ESI) : m/z [M-H]¹⁻ 295; Rt = 10.28 min.

### Example 15

### 2-(3-{[4-(morpholin-4-yl)phenyl]amino}phenyl)N-(pyrimidin-4-yl)propanamide

MS (ESI): m/z [M-H]¹⁻ 402; Rt = 10.30 min.

### Example 16

### 2-{4-[(2,6-dichloro-3-methylphenyl)amino]phenyl}-N-[2-(morpholin-4-yl)ethyl]propanamide

MS (ESI): m/z [M+H]¹⁺ 436; Rt = 9.70 min.

### Example 17

### 2-{4-[(2,6-dichloro-3-methylphenyl)amino]phenyl}-N[2-(cyclohexylamino)propyl] propanamide;

MS (ESI) : m/z [M+H]¹⁺ 460; Rt = 9.72 min.

### Example 18

### N-(2-amino-2-methylpropyl)-2-{3-[3-(trifluoromethaxy)phenaxy] phenylpropanamide MS (ESI): m/z [M+H]¹⁺ 397; Rt = 9.73 min.

### Example 19

### N-[(2-pyrrolidin-1-yl)ethyl]-2-{3-[3-{trifluoromethoxy)phenoxy]phenyl}propanamide

MS (ESI) m/z [M+H]¹⁺ 423; Rt = 9.72 min.

### Example 20

### 3-(1-{[2-(4-fluorophenoxy)ethyl] amino}-1-oxopropan-2-yl)phenyl trifluoromethanesulfonate

MS (ESI) : m/z [M+H]¹⁺ 436 Rt = 1.70 min.

### Example 21

### 2-{4-[(propan-2-ylsulfonyl)amino]Phenyl}-N(4-tert-butyl-1,3-thiazol-2-yl) propanamide

MS (ESI): m/z [M+H]¹⁺ 410 Rt = 1.68 min.

### Example 22

### N-{2-[(3-methoxyhenzyl)(methyl)amino]ethyl}-2-{4-[(propan-2-ylsulfonyl)amino] phenylpropanamide

MS (ESI) : m/z [M+H]¹⁺ 462 Rt = 1.51 min.

### Example 23

### N-(2-methylprop-2-en-1-yl)-2-[3-(thiophen-2-ylcarbonyl)phenyl]propanamide

MS (ESI) : m/z [M+H]¹⁺ 314; Rt = 10.35 min.

### Example 24

### N-(1,3-dimethyl]-1H-pyirazol-5-yl)-2-[3-(thiophen-2-ylcarbonyl)phenyl] propanamide

**MS (ESI) : m/z [M+H]¹⁺ 354; Rt = 10.12 min.**

### Example 25

### 2-{4-[(2,3-dimethoxyphenyl)amino]phenyl}-N-(1,3-dimethyl-1H-pyrazol-5-yl) propanamide

MS (ESI): m/z [M+H]¹⁺ 395; Rt = 10.73 min.

### Example 26

### 2-{4-[(2,3-dimethoxyphenyl)amino]phenyl}-N-(pyrimidin-4-yl)propanamide

MS (ESI) : m/z [M+H] ¹⁺ 379; Rt = 10.91 min.

### Example 27

### 2-{3-[hex-1-en-1-yl]phenyl}-N-[2-(propan-2-ylamnino)ethyl]propanamide

MS (ESI): m/z [M+H] ¹⁺ 317; Rt = 10.05 min.

### Example 28

### 2-{3-[hex-1-en-1-yl]phenyl}-N-(pyrimidin-4-yl)propanamide

MS (ESI) : m/z [M+H]¹⁺ 309; Rt = 12.86 min.

### Example 29

### N-(3-ethyl-1H-pyrazol-5-yl)-2-(4-{[4-(trifluorometltyl)-1,3-oxazol-2-yl]amino}phenyl) propanamide

MS (ESI): m/z [M+H]¹⁺ 408; Rt = 1.28 min.

### Example 30

### N-[2-(tert-butylamino)-2-oxoethyl]-2-(4-{[4-(trifluoromethyl)-1,3-oxazol-2-yl]amino} phenyl)propanamide

MS (ESI): m/z [M+H] ¹⁺ 413; Rt = 1.24 min.

### Example 31

### N-{2-[(3-methoxyl)(phenzyl)(methyl)amino]ethyl}-2-(4-{[4-(trifluoromethyl}-1,3-oxozol-2-yl] amino} phenyl)propanamide

MS (ESI) : m/z [M+H]¹⁺ 491; Rt = 1.30 min.

### Example 32

### N-[2-hydroxy-3-(3)4-dimethylphenoxy)propyl]-2-(4-{[4-(trifluoromethyl)-1,3-oxazol-2-yl] amino}phenyl)propanamide

MS (ESI): m/z [M+H]¹⁺ 478; Rt = 1.68 min.

### Example 33

### 2-[3-(phenylcarbonyl)l)phenyl]-N-(1,3-thiazol-2-yl)propanamide

¹H-NMR (CDCl₃) δ 7.90-7.85 (m, 3H), 7.75 (m, 1H), 7.65-7.55 (m, 2H), 7.52-7.45 (m, 3H), 7.40 (d, 1H, J = 3Hz), 6.95 (d, 1H, J = 3Hz), 3.95 (q, 1H, J = 7Hz), 1.70 (d, 3H, J = 7Hz).

### Example 34

### N-cyclohexyl-2-[3-(phenylcarbonyl)phetiyl]propanamide

MS (ESI): m/z [M+H]¹⁺ 336; Rt = 11.41 min.

### Example 35

### N-phenyl-2-[3-(phenylcarbonyl)phenyl]propanamide

MS (ESI) : m/z [M+H]¹⁺ 330; Rt = 11.28 min.

### Example 36

### N-(1,3-dimethyl-1H-pyrazol-5-yl)-2-[3-(phenylcarbonyl)phenyl] propanamide

MS (ESI): m/z [M-H]¹⁻ 346; Rt = 10.30 min.

### Example 37

### 2-[4-(2-methylpropyl)phenyl]-N-(pyridin-4-yl)propanamide

¹H-NMR (CDCl₃) δ 8.45 (d, 2H, J = 3Hz), 7.40 (d, 2H, J = 3Hz), 7.25 (d, 2H, J = 7Hz), 7.10 (d, 2H, J = 7Hz), 7.05 (bs, 1H, CONH), 3.75 (q, 1H, J = 7Hz), 2.50 (d, 2H, J = 7Hz), 1.90 (m, 1H), 1.60 (d, 3H, J = 7Hz), 0.90 (d, 6H, J = 7Hz).

### Example 38

### N-carbamoyl-2-[4-(2-methylpropyl)phenyl]propanamide

¹H-NMR (CDCl₃) δ 8.19-8.03 (bs, 2 H, CONH), 7.25 (d, 2H, J = 7Hz), 7.10 (d, 2H, J = 7Hz), 5.25 (bs, 1H, CONH), 3.64 (q, 1H, J = 7Hz), 2.46 (d, 2H, J = 7Hz), 1.86 (m, 1H), 1.53 (d, 3H, J = 7Hz), 0.91 (d, 6H, J = 7Hz).

### Example 39

### 1-methyl-4-({2-[4-(2-methylpropyl)phenyl]propanoyl}amino)pyrimidin-1-ium iodide

¹H-NMR (DMSO-d₆) δ 9.35 (s, 1H), 8.93 (d, 1H, J = 7Hz), 8.45 (d, 1H, J = 7Hz), 7.32 (d, 2H, J = 7Hz), 7.14 (d, 2H, J = 7Hz), 4.10 (q, 1H, J = 7Hz), 4.06 (s, 3H), 2.42 (d, 2H, J = 7Hz), 1.81 (m, 1H), 1.44 (d, 3H, J = 7Hz), 0.85 (d, 6H, J = 7Hz).

### Example 40

### N-{1,3-dimethyl-1H-pyrazol-5-yl)-2-[4-(2-methylpropyl)phenyl] propanamide

MS (ESI): m/z [M+H]¹⁺ 300; Rt = 11.60 min.

### Example 41

### N-(1-ethyl-3-methyl-1H-pyrazol-5-yl)-2-(4-{[4-(trifluoromethyl)-1,3-thiazol-2-yl]amino}phenyl)propanamide

MS (ESI): m/z [M+H]¹⁺ 424; Rt = 1.59 min.

### Example 42

### N-[2-(3,5-dimethylpiperidin-1-yl)ethyl]-2-(4-{[4-(trifluoromethyl)-1,3-thiazol-2-yl]amino}phenyl)prapanamide

MS (ESI): m/z [M+H]¹⁺ 455; Rt = 1.73 min.

### Example 43

### N[furan-2-yl(morpholin-4-yl)methyl]-2-(4-{[4-(trifluoromethyl)-1,3-thiazol-2-yl]amino}phenyl)propanamide

MS (ESI): m/z [M+H]¹⁺ 495; Rt = 1.65 min.

### Example 44

### N-[4-(pyridin-4-ylmethyl)phenyl]-2-(4-{[4-(trifluoromethyl)-1,3-thiazol-2-yl]amino} phenyl)propanamide

MS (ESI): m/z [M+H]¹⁺ 483; Rt = 1.66 min.

### Example 45

### N-[2-(furan-2-yl)propyl]-2-(4-{[4-(trifluomethyl)-1,3-thiazol-2-y]amino}phenyl) propanamide

MS (ESI): m/z [M+H]¹⁺ 424; Rt = 1.68 min.

### Example 46

### 4-(1-{[2-(furan-2-yl)propyl]amino}-1-oxopropan-2-yl)phenyl trifluoromethanesulfonate

MS (ESI): m/z [M+H]¹⁺ 483; Rt = 1.66 min.

### Example 47

### 4-[1-oxo-1-(Pyridin-4-ylamino)prapan-2-yl]phenyl trifluoromethanesulfonate

¹H-NMR (CDCl₃) δ 8.45 (d, 2H, J = 7Hz), 7.45 (m, 4H), 7.30 (d, 2H, J = 7Hz), 3.75 (q, 1H, J = 7Hz), 1.60 (d, 3H, J = 7Hz).

### Example 48

### 4-{1-oxo-1-[4-(pyridin-4-ylmethyl)propan-2-yl] amino}phenyl trifluoromethanesulfonate

MS (ESI): m/z [M+H]¹⁺ 465; Rt = 1.30 min.

### Example 49

### 4-(1-{[(dimethylamino)(4-fluorophenyl)methyl]amino}-1-oxopropan-2-yl)phenyl trifluoromethanesulfonate

MS (ESI): m/z [M+H]¹⁺ 463; Rt= 1.31 min.

### Example 50

### 4-(1-{[3-[3-methoxybenzyl(methyl)amino]propyl]amino-1-oxopropan-2-yl)phenyl trifluoromethanesulfonate

MS (ESI): m/z [M+H]¹⁺ 489; Rt = 1.33 min.

### Example 51

### 4-[3-(3,4-dimethylphenoxy)-2-hydroxypropyl]amino-1-oxopropan-2-yl)phenyl trifluoromethanesulfonate

MS (ESI): m/z [M+H] ¹⁺ 476; Rt = 1.73 min.

### Example 52

### 2-(3- {[3-methoxy-5-(trifluoromethyl)phenyl]amino}phenyl)-N-(3-ethoxypropyl) propanamide

MS (ESI): m/z [M+H]¹⁺425; Rt = 11.91 min.

### Example 53

### 2-(3-{[3-methoxy-5-(trifluoromethyl)phenyl]amino}phenyl)-N-(1H-pyrrol-1-yl) propanamide

MS (ESI): m/z [M+H]¹⁺ 404; Rt = 11.62 min.

### Example 54

### N'-{2-[3-(3-methoxy-5-(trifluoromethyl)phenylamino)phenyl]propanoyl}furan-2-carbohydrazide

MS (ESI): m/z [M+H]¹⁺ 439; Rt = 11.49 min.

### Example 55

### 2-(3-{[3-methoxy-5-(trifluoromethyl)phenyl]amino}pbenyl)-N-(pyrimidin-4-yl) propanamide

MS (ESI): m/z [M+H]¹⁺ 417; Rt = 11.78 min.

### Example 56

### N-ethyl-2-(3-{[3-methoxy-5-(trifluoromethyl)phenyl]amnino} phenyl)propanamide

MS (ESI): m/z [M+H]¹⁺ 367; Rt = 11.51 min.

### Example 57

### 2-{3-[(3-methoxy-5-(trifluoromethyl)phenyl)amino]phenyl}-N-[2-(benzylamino)ethyl]propanamide

MS (ESI): m/z [M+H]1⁺ 472; Rt = 10.05 min.

### Example 58

### N-(2-amino-2-methylpropyl)-2-[3-{[3-methoxy-5-(trifluoromethyl)phenyl] amino}phenyl]propanamide

MS (ESI): m/z [M+H]¹⁺ 410; Rt = 9.71 min.

### Example 59

### N-(2-aminocyclohexyl)-2-[3-{[3-methoxy-5-(trifluoromethyl)phenyl] amino}phenyl]propanamide

MS (ESI): m/z [M+H]¹⁺ 436; Rt = 9.98 min.

### Example 60

### methyl 3-[(tert-butoxycarbonyl)amino)-2-[4-(naphthalen-1-yloxyphenyl)propanoyl] aminopropanoate

MS (ESI): m/z [M+H]¹⁺ 493; Rt = 12.65 min.

### Example 61

### N-[2-(benzylamino)ethyl]-2-[4-(naphthalen-1-yloxy)phenyl]propanamide

MS (ESI): m/z [M+H]¹⁺ 425; Rt = 10.23 min.

### Example 62

### N-[3-(dimethylamino)propyl]-2-[4-(naphthalen-1-yloxy)phenyl]propanamide

MS (ESI): m/z [M+H]¹⁺ 377; Rt = 9.77 min.

### Example 63

### N-[3-(cyclohexylamino)propyl]-2-[4-(naphthalen-1-yloxy)phenyl]propanamide

MS (ESI): m/z [M+H]¹⁺ 431; Rt = 10.45 min.

### Example 64

### 2-[4-(naphthralen-1-yloxy)phenyl]-N-(4H-1,2,4-triazol-4-yl)propanamide

MS (ESI): m/z [M+H]¹⁺ 359; Rt = 11.30 min.

### Example 65

### 2-[4-(naphthalen-1-yloxy)phenyl]-N-[2-(1-methylpyrrolidin-2-yl)ethyl] propanamide

MS (ESI): m/z [M+H]¹⁺ 403; Rt = 9.82 min.

### Example 66

### N-[2-(acetylamino)ethyl]-2-[4-(naphthalen-1-yloxy)phenyl]propanamide

MS (ESI): m/z [M+H]¹⁺ 377; Rt = 11.50 min.

### Example 67

### 2-[4-(naphthalen-1-yloxy)phenyl]-N-[2-(morpholin-4-yl)ethyl] propanamide

MS (ESI): m/z [M+H]¹⁺ 405; Rt = 10.28 min.

### Biological Evaluation

Calcium mobilization assay (FLIPR) - Human (IMR-90) lung fibroblast cells expressing native B1 receptors were harvested by trypsinization and seeded into black wall/clear bottom 96-well plates (Costar 3904; Corning Life Sciences, Acton, MA) at approximately 13,000 cells/well. After 1 day incubation, cells were treated with human IL-1β (0.35 ng/ml) in 10% FBS/MEM for 2 h to up-regulate BKB 1 receptors. Induced cells were loaded with fluorescent calcium indicator by incubation with 2.3 uM Fluo-4/acetoxymethyl ester (Invitrogen) at 37°C for 1.5 h in the presence of an anion transport inhibitor (2.5 mM probenecid in 1% FBS/MEM). Extracellular dye was removed by washing with assay buffer (2.5 mM probenecid, 0.1% BSA in 20 mM HEPES/HBSS without bicarbonate or phenol red, pH 7.5) and cell plates were kept in the dark until used. Test compounds were assayed at eight concentrations in triplicate. Addition of test compounds to the cell plate and incubation for 5 min at 35°C, followed by the addition of 2 to 8 nM final BK1 agonist *des*Arg¹⁰-kallidin (DAKD, 3 x EC₅₀) was carried out in the fluorimetric imaging plate reader (FLIPR; Molecular Devices) while continuously monitoring calcium-dependent fluorescence.

Responses for B2 receptors were measured in IMR-90 cells in an identical manner except that IL-1βtreatment was not necessary and bradykinin (0.7 nM final; 3 x EC₅₀) replaced DAKD as agonist.

The compounds 1-82 of the invention were tested in the above described assay and found active in inhibiting calcium mobilization in the pIC₅₀ range of 4-7 in BKB1 receptors-expressing cells, while the same compounds were found inactive in BKB2 receptors-expressing cells biological assay.

### List of the preferred compounds

| N. | Chemical Structure | Chemical Name |
|---|---|---|
| 1 | 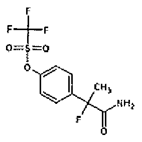 | 4-(1-amino-2-fluoro-1-oxopropan-2-yl)phenyl trifluoromethanesulfonate |
| 2 | 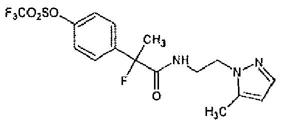 | 4-(2-fluoro-1-{[2-(5-methyl-1*H*-pyrazol-1-yl)ethyl]amino}-1-oxopropan-2-yl)phenyl trifluomethanesulfonate |
| 3 | 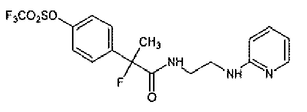 | 4-(2-fluoro-1-oxo-1-{[2-(pyridin-2-ylamino)ethyl]amino}propan-2-yl)phenyl trifluoromethanesulfonate |
| 4 | 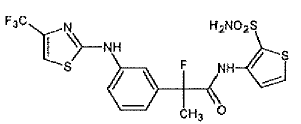 | 2-fluro-*N*-(2-sulfamoylthiophen-3-yl)-2-(3-{[4-(trifluoromethyl)-1,3-thiazol-2-yl]amino}phenyl)propanamide |
| 5 | 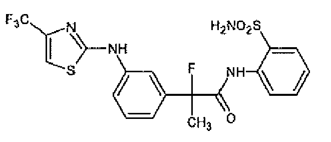 | 2-fluoro-*N*-(2-sulfamoylphenyl)-2-(3-{[4-(trifluoromethyl)-1,3-thiazol-2-yl]amino}phenyl)propanamide |
| 6 | 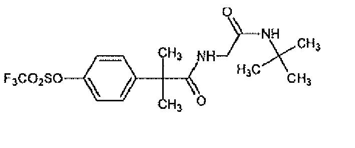 | 4-(2-methyl-1-{[2-(*tert*-butylamino)-2-oxoethyl]amino}-1-oxopropan-2-yl)phenyl trifluoromethanesulfonate |
| 7 | 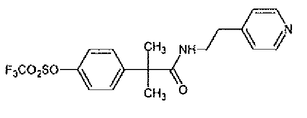 | 4-(2-methyl-1-oxo-1-{[2-(pyridin-4-yl)ethyl]amino}propan-2-yl)phenyl trifluoromethanesulfonate |
| 8 | 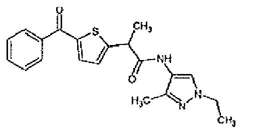 | *N*-(1-ethyl-3-methyl-1*H*-pyrazol-4-yl)-2-[5-(phenylcarbonyl)thiophen-2-yl]propanamide |
| 9 | 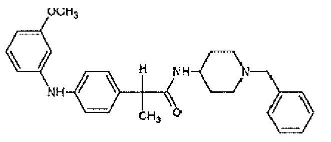 | 2-{4-[(3-methoxyphenyl)amino]phenyl}-*N*-(1-benzylpiperidin-4-yl)propanamide |
| 10 | 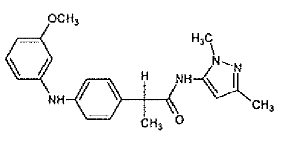 | 2-[(3-methoxyphenyl)amino]phenyl}-*N-*(1,3-dimethyl-1*H*-pyrazol-5-yl) propanamide |
| 11 | 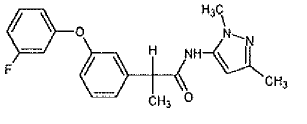 | *N*-(1,3-dimethyl-1*H*-pyrazol-5-yl)-2-[3-(3-fluorophenoxy)phenyl] propanamide |
| 12 | 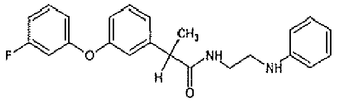 | 2-[3-(3-fluorophenoxy)phenyl]-*N*-[2-(phenylamino)ethyl]propanamide |
| 13 | 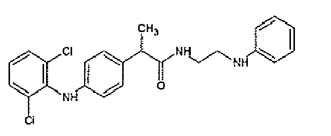 | 2-{4-[(2,6-dichlorophenyl)amino]phenyl}-*N*-phenylpropanamide |
| 14 | 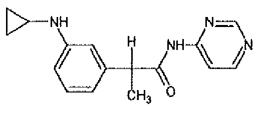 | 2-[3-(cyclopropylamino)phenyl]-*N*(pyrimidin-4-yl)propanamide |
| 15 | 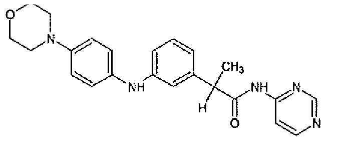 | 2-(3-{[4-(morpholin-4-yl)phenyl]amino}phenyl)N-(pyrimidin-4-yl)propanamide |
| 16 | 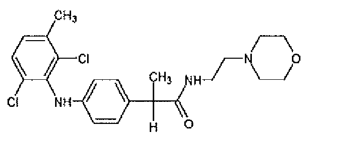 | 2-{4-[(2,6-dichloro-3-methylphenyl)amino]phenyl}-*N*-[2-(morpholin-4-yl)ethyl] propanamide |
| 17 | 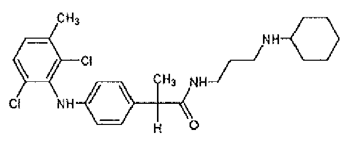 | 2-{4-[(2,6-dichloro-3-methylphenyl}amino]phenyl}-*N*-[2-(cyclohexylamino)propyl]propanamide |
| 18 | 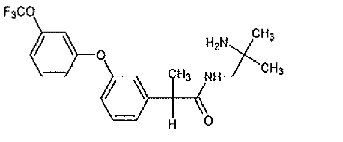 | *N*-(2-amino-2-methylpropyl)-2-{3-[3-(trifluoromethoxy)phenoxy]phenylpropanamide |
| 19 | 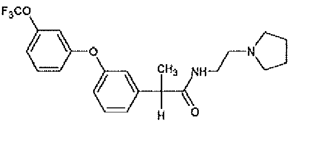 | *N*-[(2-pyrrolidin-1-yl)ethyl]-2-{3-[3-(trifluoromethoxy)phenoxy]phenyl}propanamide |
| 20 | 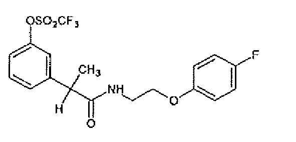 | 3-(1-{[2-(4-fluorophenoxy)ethyl]amino]-1-oxopropan-2-yl)phenyltrifluoromethanesulfonate |
| 21 | 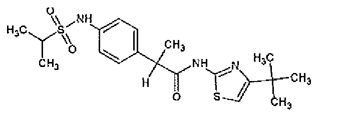 | 2-{4-[(propan-2-ylsulfonyl)amino]phenyl}-*N*-(4-tert-butyl-1,3-thiazol-2-yl)propanamide |
| 22 | 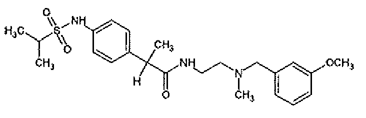 | *N*-{2-[(3-methoxybenzyl)(methyl)amino]ethyl} -2-(4-[(propan-2-ylsulfonyl)amino] phenylpropanamide |
| 23 | 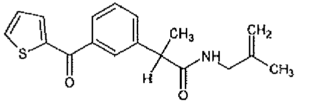 | *N*-(2-methylprop-2-en-1-yl)-2-[3-(thiophen-2-ylcarbonyl)phenyl]propanamide |
| 24 | 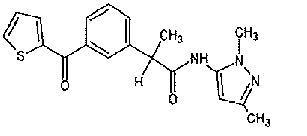 | *N*-(1,3-dimethyl-1*H*-pyrazol-5-yl)-2-[3-(thiophen-2-ylcarbonyl)phenyl] propanamide |
| 25 | 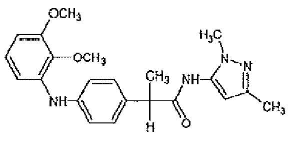 | 2-{4-[(2,3-dimethoxyphenyl)amino]phenyl}-*N*-(1,3-dimethyl-1*H*-pyrazol-5-yl)propanamide |
| 26 | 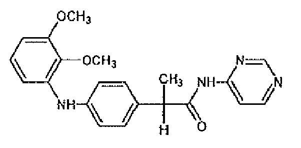 | 2-{4-[(2,3-dimethoxyphenyl)amino]phenyl}-*N* (pyrimidin-4-yl)propanamide |
| 27 | 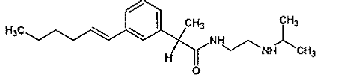 | 2-{3-[hex-1-en-1-yl]phenyl}-*N*-[2-(propan-2-ylamino)ethyl]propanamide |
| 28 | 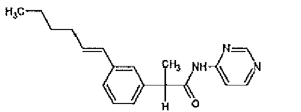 | 2-{3-[hex-1-en-1-yl]phenyl}-*N*-(pyrimidin-4-yl)propanamide |
| 29 | 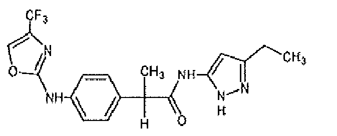 | *N*-(3-ethyl-1*H*-pyrazol-5-yl)-2-(4-{[4-(trifluoromethyl)-1,3-oxazol-2-yl]amino} phenyl)propanamide |
| 30 | 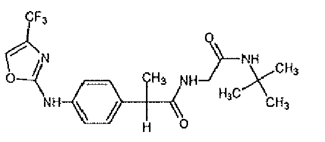 | *N*-[2-(tert-butylamino)-2-oxoethyl]-2-(4-{[4-(trifluoromethyl)-1,3-oxazol-2-yl]amino} phenyl)propanamide |
| 31 | 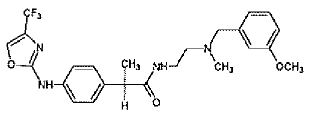 | *N*-{2-[(3-methoxybenzyl)(methyl)amino]ethyl}-2-(4-{[4-(trifluoromethyl)-1,3-oxazol-2-yl]amino}phenyl}propanamide |
| 32 | 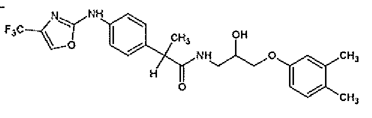 | *N*-[2-Hydroxy-3-(3,4-dimethylphenoxy)propl]-2-(4-{{[4-(trifluorometyl)-1,3-oxazol-2-yl]amino}phenyl)propanamide |
| 33 | 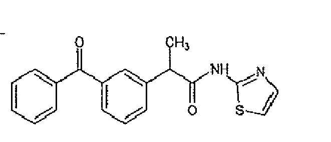 | 2-[3-(phenylcarbonyl)phenyl]-*N*-(1,3-thiazol-2-yl)propanamide |
| 34 | 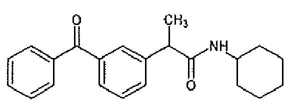 | *N*-cyclohexyl-2-[3-(phenylcarbonyl)phenyl]propanamide |
| 35 | 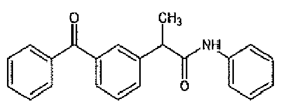 | *N*-phenyl-2-[3-(phenylcarbonyl)phenyl]propanamide |
| 36 | 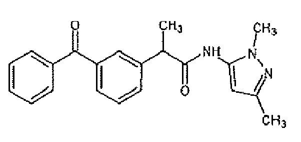 | *N*-(1,3-dimethyl-1*H*-pyrazol-5-yl)-2-[3-(phenylcarbononyl)phenyl]propanamide |
| 37 | 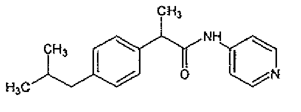 | 2-[4-(2-methylpropyl)phenyl]-*N*-(pyridin-4-yl)propanamide |
| 38 | 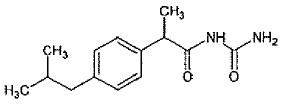 | *N*-carbamoyl-2-[4-(2-methylpropyl)phenyl]propanamide |
| 39 | 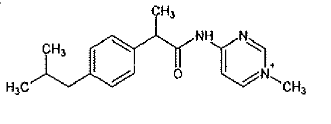 | 1-methyl-4-({2-[4-(2-methylpropyl)phenyl]propanoyl}amino)pyrimidin-1-ium iodide |
| 40 | 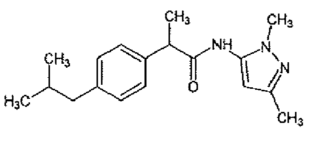 | *N*-(1,3-dimethyl-1*H*-pyrazol-5-yl)-2-[4-(2-methylpropyl)phenyl] propanamide |
| 41 | 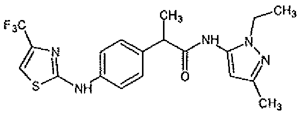 | *N*-(1-ethyl-3-methyl-1*H*-pyrazol-5-yl)-2-(4-{[4-(trifluoromethyl)-1,3-thiazol-2-yl]amino) phenyl)propanamide |
| 42 | 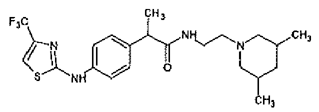 | *N*-[2-(3,5-dimethylpiperidin-1-yl)ethyl]-2-(4-{[4-(trifluoromethyl)-1,3-thiazol-2-yl]amino}phenyl)propanamide |
| 43 | 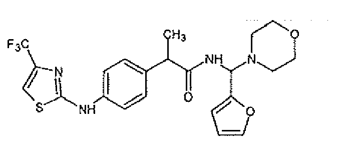 | *N*-[furan-2-yl(morpholin-4-yl)methyl]-2-(4-{[4-(trifluoromethyl)-1,3-thiazol-2-yl]amino} phenyl)propanamide |
| 44 | 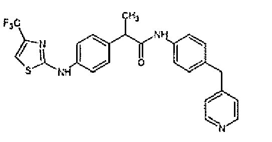 | *N*-[4-(pyridin-4-ylmethyl)phenyl]-2-(4-{[4-(trifluoromethyl)-1,3-thiazol-2-yl]amino}phenyl) propanamide |
| 45 | 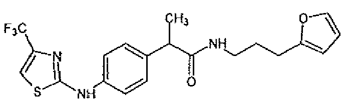 | *N*-[2-(furan-2-yl)propyl]-2-(4-{[ 4-(trifluoromethyl)-1,3-thiazol-2-yl]amino}phenyl)propanamide |
| 46 | 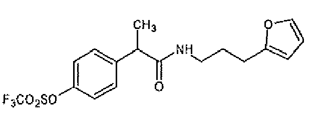 | 4-(1-{[2-(furan-2-yl)propyl]amino}-1-oxopropan-2-yl)phenyl trifluoromethanesulfonate |
| 47 | 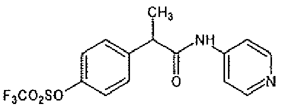 | 4-[1-oxo-1-(pyridin-4-ylamino)propan-2-yl]phenyl trifluoromethanesulfonate |
| 48 | 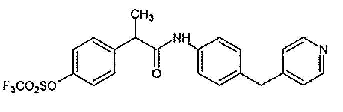 | 4-{1-oxo-1-[4-(pyridin-4-ylmethyl)propan-2-yl]amino}phenyl trifluoromethanesulfonate |
| 49 | 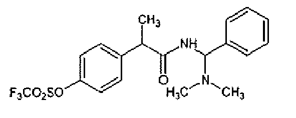 | 4-(1-{[(dimethylamino)(4-fluorophenyl)methyl]amino}-1-oxopropan-2-yl)phenyl trifluoromethanesulfonate |
| 50 | 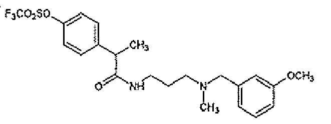 | 4-(1-{[3-[3-methoxybenzyl(methyl)amino]propyl]amino-1-oxopropan-2-yl)phenyl trifluoromethanesulfonate |
| 51 | 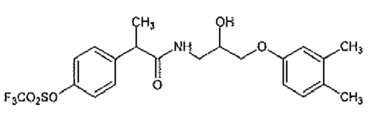 | 4-[3-(3,4-dimethylphenoxy)-2-hydroxypropyl]amino-1-oxopropan-2-yl)phenyl trifluoromethanesulfonate |
| 52 | 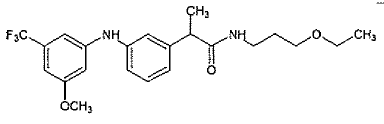 | 2-(3-{[3-methoxy-5-(trifluoromethyl)phenyl]amino} phenyl)-*N*-(3-ethoxypropyl) propanamide |
| 53 | 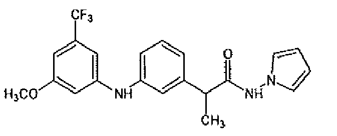 | 2-(3-{[3-methoxy-5-(trifluoromethyl)phenyl]amino}phenyl)-*N*-(1*H*-pyrrol-1-yl) propanamide |
| 54 | 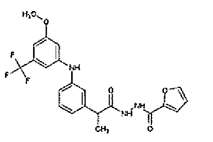 | *N*'-{2-[3-(3-methoxy-5-(trifluoromethylamino)phenyl] propanoyl}furan-2-carbohydrazide |
| 55 | 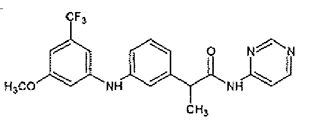 | 2-(3-{[3-methoxy-5-(trifluoromethyl)phenyl]amino} phenyl)-*N*-(pyrimidin-4-yl)propanamide |
| 56 | 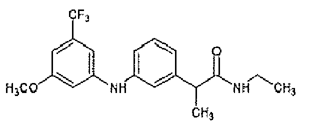 | *N*-ethyl-2-(3-{[3-methoxy-5-(trifluoromethyl)phenyl]amino} phenyl)propanamide |
| 57 | 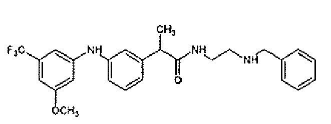 | 2-{3-[(3-methoxy-5-(trifluoromethyl)phenyl)amino]phenyl}-*N*-[2-(benzylamino)ethyl]propanamide |
| 58 | 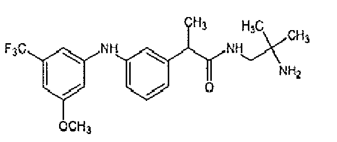 | *N*-(2-amino-2-methylpropyl)-2-[3-{[3-methoxy-5-(trifluoromethyl)phenyl] amino}phenyl]propanamide |
| 59 | 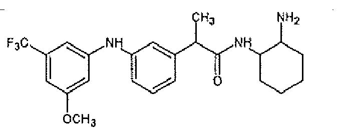 | *N*-(2-aminocyclohexyl)-2-[3-{[3-methoxy-5-(trifluoromethyl)phenyl] amino} phenyl]propanamide |
| 60 | 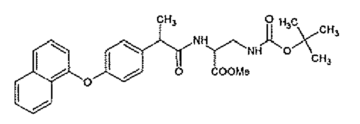 | methyl 3-[(tert-butoxycarbonyl)amino]-2-[4-(naphthalen-1-yloxyphenyl) propanoyl] aminopropanoate |
| 61 | 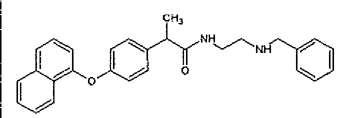 | *N*-[2-(benzylamino)ethyl]-2-[4-(naphthalen-1-yloxy)phenyl]propanamide |
| 62 | 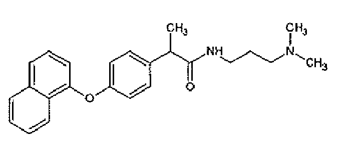 | *N*-[3-(dimethylamino)propyl]-2-[4-(naphthalen-1-yloxy)pheny)]propanamide |
| 63 | 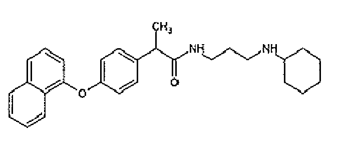 | *N*-[3-(cyclohexylamino)propyl]-2-[4-(naphthalen-1-yloxy)phenyl]propanamide |
| 64 | 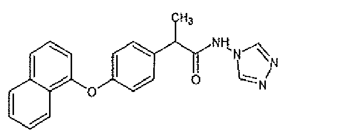 | 2-[4-(naphthalen-1-yloxy)phenyl]-*N*-(4*H*-1,2,4-triazol-4-yl)propanamide |
| 65 | 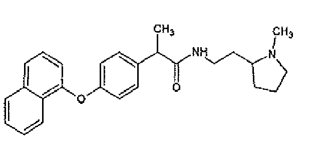 | 2-[4-(naphthalen-1-yloxy)phenyl]-*N*-[2-(1-methy)pyrroldin-2-yl)ethyl]propanamide |
| &6 | 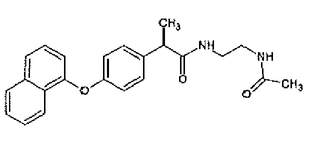 | *N*-[2-(acetylamino)ethyl]-2-[4-(naphthalen-1-yloxy)phenyl]propanamide |
| 67 | 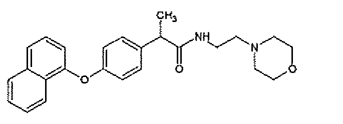 | 2-[4-(naphthalen-1-yloxy)phenyl]-*N*-[2-(morpholin-4-yl)ethyl]propanamide |
| 69 | 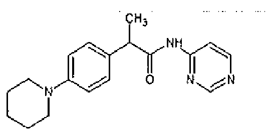 | 2-[4-(piperidin-1-yl)phenyl]-*N*(pyrimidin-4-yl)propanamide |
| 70 | 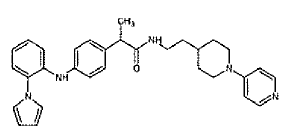 | *N*-{2-[1-(pyridin-4-yl)piperidin-4-yl]ethyl}-2-(4-{[2-(1*H*-pyrrol-1-yl)phenyl]amino}phenyl) propanamide |
| 71 | 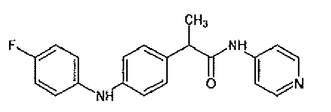 | 2-{4-[(4-fluorophenyl)amino]phenyl}-*N*-(pyridin-4-yl)propanamide |
| 72 | 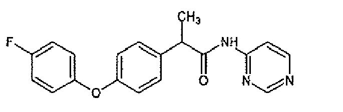 | 2-[4-(4-fluorophenoxy)phenyl]-*N*-(pyrimidin-4-yl)propanamide |
| 73 | 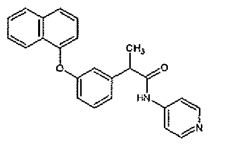 | 2-[3-(naphthalen-1-yloxy)phenyl]-*N*-(pyridin-4-yl)propanamide |
| 74 | 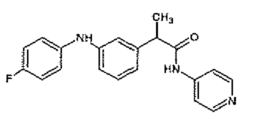 | 2-{3-[(4-fluorophenyl)amino]phenyl}-*N*-{pyridin-4-yl)propanamide |
| 75 | 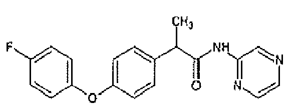 | 2-[4-(4-fluorophenoxy)phenyl]-*N*-(pyrazin-2-yl)propanamide |
| 76 | 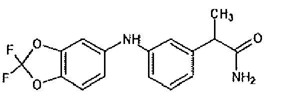 | 2-{3-[(2,2-difluoro-1,3-benzodioxol-5-yl)amino]pheyl}propanamide |
| 77 | 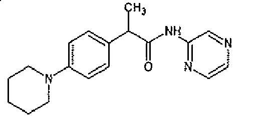 | 2-[4-(piperidin-1-yl)phenyl]-*N*-(pyrazin-2-yl)propanamide |
| 78 | 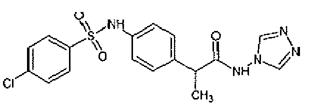 | 2-(4-{[(4-chlorophenyl)sulfonyl]amino}phenyl)-*N-*(4*H*-1,2,4-triazol-4-yl)propanamide |
| 79 | 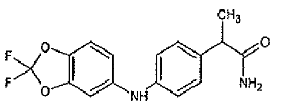 | 2-{4-[(2,2-difluoro-1,3-benzodioxol-5-yl)amino]phenyl}propanamide |
| 80 | 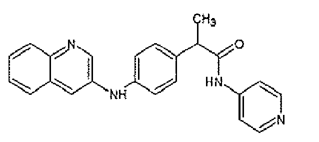 | *N*-(pyridin-4-yl)-2-[4-(quinolin-3-ylamino)phenyl]propanamide |
| 81 | 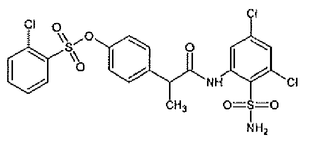 | 4-{1-[(3,5-dichloro-2-sulfamoylphenyl)amino]-1-oxopropan-2-yl}phenyl 2-chlorobenzenesulfonate |
| 82 | 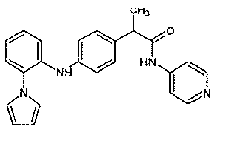 | 2-[4-{[2-(1H-pyrrol-1-yl)phenyl]-N-(pyridin-4-yl)propanamide |

## Claims

1. Compounds of formula (I): 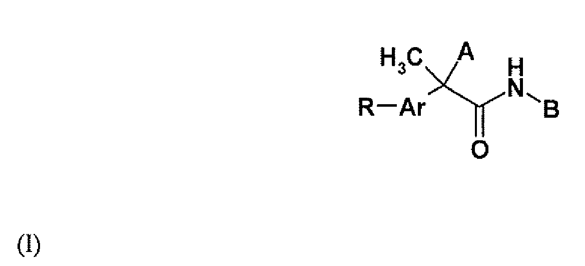 and pharmaceutically acceptable salts thereof,
wherein
A is selected from the group consisting of H, CH₃ and F;
Ar is a selected from the group consisting of phenyl and 5, 6-membered heteroatyl;
R is a residue selected from the group consisting of:
- linear or branched C₁-C₆-alkyl or C₂-C₈-alkenyl, C₁-C₄-aminoalkyl,
- 3-6 membered cycloalkylamino;
- W-Ar₁ wherein W is selected from O, NH, CO and Ar₁ is selected from the group consisting of optionally substituted phenyl, naphtyl, quinolinyl, benzodioxolyl and 5-6-membered heteroaryl;
- optionally substituted 5 -6-membered heterocyclic residues; and
- X-SO₂R₁, wherein X is selected from NH and O and R1 is selected from linear or branched C₁-C₄ alkyl, C₁-C₄ haloalkyl and optionally substituted phenyl;
B is a residue selected from the group consisting of:
- H, linear or branched C₁-C₆ alkyl, C₂-C₈-alkenyl, C₁-C₄ alkylamino, carbamoyl;
- (CH₂)ₙ-(NH)ₚ-Y wherein n is between 0 and 3, p is 0 or 1 and Y is selected from:
- a 5-6 membered ring selected from optionally substituted phenyl, heteroaryl, cycloalkyl and heterocyclic residues;
- benzyl, 5-6 membered heteroarylcarbonyl, C₁-C₆-alkyl, linear or branched C₁-C₃-alkylcarbonyl, C₁-C₆-alkoxy and C₁-C₆-alkoxy hydroxy substituted.
- (CH₂)ₙ-Z-(CH2)_{n'}-A wherein n is between 0 and 3, n' is between 0 and 1, Z is selected from -CONH-, -O-, -NCH₃-, -CHOH- and A is selected from linear or branched C₁-C₄ alkyl, substituted or unsubstituted phenyl, substituted or unsubstituted phenoxy;
- CHRₐR_{b}, wherein Rₐ and R_{b} are independently selected from substituted or unsubstituted 5-6 membered heteroaryl, substituted or unsubstituted 5-6 membered heterocyclic, substituted or unsubstituted phenyl, dialkylamino, -CH₂-NHCOO-C₁-C₄-alkyl, -(COO)C₁-C₄-alkyl.

2. Compounds as claimed in claim 1, wherein Ar is selected from substituted or unsubstituted phenyl, tiophene and pyrrole;

3. Compounds as claimed in claim 2, wherein when said Ar is phenyl, R is in position 3 or 4 of said Ar;

4. Compounds as claimed in claim 2, wherein said Ar is thiopen-2-yl.

5. Compounds as claimed in claims 1 to 4, wherein R is selected from hex-1-en-1-yl, 2-methylpropyl, cyclopropylamino, substituted or unsubstituted phenylcarbonyl, substituted or unsubstituted tiophen-carbonyl, substituted or unsubstituted phenylamino, substituted or unsubstituted 1,3-thiazol-2-yl-amino, substituted or unsubstituted 1,3-oxazol-2-yl-amino, substituted or unsubstituted phenoxy, substituted or unsubstituted naphtalen-1-yloxy, substituted or unsubstituted naphtalen-2-yloxy morpholin-4-yl, pyperidin-1-yl, trifluoromethanesulfonyloxy, C₁-C₄ alkylsulfonylamino, substituted or unsubstituted phenylsulfonylamino, substituted or unsubstituted phenylsulfonyloxy.

6. Compounds as claimed in claims 1 to 5, wherein B is selected from H, ethyl, 2-methylprop-2-en-1-yl, 2-amino-2-methyl-propyl, substituted or unsubstituted 1*H*-pyrazol-4-yl, substituted or unsubstituted 1*H*-pyrazol-5-yl, substituted or unsubstituted tiophen-3-yl, substituted or unsubstituted 1,3-thiazol-2-yl, pyrimidin-4-yl, substituted or unsubstituted 1-*H*-pyrrol-1-yl, substituted or unsubstituted 4*H*-1,2,4-triazol-4-yl, substituted or unsubstituted pyridine-4-yl, pyrazin-2-yl, substituted or unsubstituted piperydin-4-yl, substituted or unsubstituted phenyl, substituted or unsubstituted ciclohexyl, furan-2-yl-C₁-C₃-alkyl, substituted or unsubstituted piperidin-1-yl-C₁-C₃-alkyl, pyridine-2-yl-amino-C₁-C₃-alkyl, phenylamino-C₁-C₃-alkyl; cyclohexylamino-N-C₁-C₃-alkyl, 1*H*-pyrazol-1-yl-C₁-C₃-alkyl, pyridin-4-yl-C₁-C₃-alkyl, morpholin-4-yl-C₁-C₃-alkyl, pyrrolidin-1-yl-C₁-C₃-alkyl, (C₁-C₆-alkylamino)-C₁-C₃-alkyl, (benzylamino)C₁-C₃-alkyl, (C₁-C₃-alkylamino)-ethyl, -(C₁-C₄-dialkylamino)C₁-C₃-alkyl, 2-(tert-butylamino)-2-axoethyl; (phenoxy)C₁-C₃alkyl, [(benzyl)(methylamino)]C₁-C₃alkyl, (3,4-dimethylphenoxy)-2-, [(dimethylamino)(4-fluorophenyl)methyl]amino; (*tert*-butoxycarbonyl) aminoetylcarboxy], carbamoyl, furan-2-carbamido.

7. Compounds as claimed in claims 1 to 6, wherein the carbon atom bound to the Ar in formula I is in R or S configuration.

8. Compounds as claimed in claim 1 selected from the group consisting of:
4-(1-amino-2-fluoro-1-oxopropan-2-yl)phenyl trifluoromethanesulfonate;
4-(2-fluoro-1-{[2-(5-methyl-1*H*-pyrazol-1-yl)ethyl]amino}-1-oxopropan-2-yl)phenyl trifluoromethanesulfonate;
4-(2-fluoro-1-oxo-1-{[2-(pyridin-2-ylamino)ethyl]amino}propan-2-yl)phenyl trifluoromethanesulfonate;
2-fluoro-*N*-(2-sulfamoylthiophen-3-yl)-2-(3-{[4-(trifluoromethyl)-1,3-thiazol-2-yl]amino}phenyl)propanamide;
2-fluoro-*N*-(2-sulfamoylphenyl)-2-(3-([4-(trifluoromethyl)-1,3-thiazol-2-yl]amino) phenyl)propanamide;
4-(2-methyl-1- {[2-(*tert*-butylamino)-2-oxoethyl]amino}-1-oxopropan-2-yl)phenyl trifluoromethanesulfonate;
4-(2-methyl-1-oxo-1-{[2-(pyridin-4-yl)ethyl]amino}propan-2-yl)phenyl trifluoromethanesulfonate;
*N*-(1-ethyl-3-methyl-1*H*-pyrazol-4-yl)-2-[5-(phenylcarbonyl)thiophen-2-yl]propanamide;
2- {4-[(3-methoxyphenyl)amino]phenyl}-*N*-(1-benzylpiperidin-4-yl)propanamide;
2-[(3-methoxyphenyl)amino]phenyl}-*N*-(1,3-dimethyl-1*H*-pyrazol-5-yl) propanamide;
*N*-(1,3-dimethyl-1*H*-pyrazol-5-yl)-2-[3-(3-fluorophenoxy)phenyl] propanamide;
2-[3-(3-fluorophenoxy)phenyl]-*N*-[2-(phenylamino)ethyl]propanamide;
2-{4-[(2,6-dichlorophenyl)amino]phenyl}-*N*-phenylpropanamide;
2-[3-(cyclopropylamino)phenyl]-N-(pyrimidin-4-yl)propanamide;
2-(3-{[4-(morpholin-4-yl)phenyl]amino}phenyl)N-(pyrimidin-4-yl)propanamide;
2-{4-[(2,6-dichloro-3-methylphenyl)amino]phenyl}-*N*-[2-(morpholin-4-yl)ethyl]propanamide;
2-{4-[(2,6-dichloro-3-methylphenyl)amino]phenyl}-*N*-[2-(cyclohexylamino)propyl] propanamide;
*N*-(2-amino-2-methylpropyl)-2-{3-[3-(trifluoromethoxy)phenoxy]phenylpropanamide;
*N*-[(2-pyrrolidin-1-yl)ethyl]-2-{3-[3-(trifluoramethoxy)phenoxy]phenyl}propanamide; 3-(1-{[2-(4-fluorophenoxy)ethyl]amino}-1-oxopropan-2-yl)phenyl trifluoromethanesulfonate;
2-{4-[(propan-2-ylsulfony])amino]phenyl}-*N*-(4-tert-butyl-1,3-thiazol-2-yl) propanamide;
*N*-{2-[(3-methoxybenzyl)(methyl)amino]ethyl}-2-{4-[(propan-2-ylsulfonyl)amino] phenylpropanamide;
*N*-(2-methylprop-2-en-1-yl)-2-[3-(thiophen-2-ylcarbonyl)phenyl] propanamide;
*N*-(1,3-dimethyl-1*H* pyrazol-5-yl)-2-[3-(thiophen-2-ylcarbonyl)phenyl] propanamide;
2-{4-[(2,3-dimethoxyphenyl}amino]phenyl}-*N*-(1,3-dimethyl-1*H*-pyrazol-5-yl) propanamide;
2-{4-[(2,3-dimethoxyphenyl)amino]phenyl}-*N*-(pyrimidin-4-yl)propanamide;
2-{3-[hex-1-en-1-yl]phenyl}-*N*-[2-(propan-2-ylamino)ethyl]propanamide;
2-{3-[hex-1-en-1-yl]phenyl}-*N*-(pyrimidin-4-yl)propanamide;
*N*-(3-ethyl-1*H*-pyrazol-5-yl)-2-(4-{[4-(trifluoromethyl)-1,3-oxazol-2-yl]amino}phenyl) propanamide;
*N*-[2-(*tert*-butylamino)-2-oxoethyl]-2-(4-{[4-(trifluoromethyl}-1,3-oxazol-2-yl]amino} phenyl)propanamide;
*N*-{2-[(3-methoxybenzyl)(methyl)amino]ethyl}-2-(4-{ [4-(trifluoromethyl)-1,3-oxazol-2-yl]amino}phenyl)propanamide;
*N*-[2-hydroxy-3-(3,4-dimethylphenoxy)propyl]-2-(4-{[4-(trifluoromethyl)-1,3-oxazol-2-yl]amino}phenyl)propanamide;
2-[3-(phenylcarbonyl)phenyl]-*N*-(1,3-thiazol-2-yl)propanamide;
*N*-cycloliexyl-2-[3-(phenylcarbotiyl)pheiiyl]propanamide;
*N*-phenyl-2-[3-(phenylcarbonyl)phenyl]propanamide;
*N*-(1,3-dimethyl-1*H*-pyrazol-5-yl)-2-[3-(phenylcarbonyl)phenyl] propanamide;
2-[4-(2-methylpropyl)phenyl]-*N*-(pyridin-4-yl)propanamide;
*N*-carbamoyl-2-[4-(2-methylpropyl)phenyl]propanamide;
1-methyl-4-({2-[4-(2-methylpropyl)phenyl]propanoyl}amino)pyrimidin-1-ium iodide;
*N*-(1,3-dimethyl-1*H*-pyrazol-5-yl)-2-[4-(2-methylpropyl)phenyl] propanamide;
*N*-(1-ethyl-3-methyl-1*H*-pyrazol-5-yl)-2-(4-{[4-(trifluoromethyl)-1,3-thiazol-2-yl]amino}phenyl)propanamide;
*N*-[2-(3,5-dimethylpiperidin-1-yl)ethyl]-2-(4-{[4-(trifluoromethyl)-1,3-thiazol-2-yl]amino}phenyl)propanamide;
*N*-[furan-2-yl(morpholin-4-yl)methyl]-2-(4-{[4(trifluoromethyl)-1,3-thiazol-2-yl]amino}phenyl)propanamide;
*N*-[4-(pyridin-4-ylmethyl)phenyl]-2-(4-{[4-(trifluoromethyl)-1,3-thiazol-2-yl]amino} phenyl)propanamide;
*N*-[2-(furan-2-yl)propyl]-2-(4-{[4-(trifluoromethyl)-1,3-thiazol-2-yl]amino}phenyl) propanamide;
4-(1-{[2-(furan-2-yl)propyl]amino}-1-oxopropan-2-yl)phenyl Trifluoromethanesulfonate;
4-[1-oxo-1-(pyridin-4-ylamino)propan-2-yl]phenyl trifluoromethanesulfonate;
4-{1-oxo-1-[4-(pyridin-4-ylmethyl)propan-2-yl]amino}phenyl trifluoromethanesulfonate;
4-(1-{[(dimethylamino)(4-fluorophenyl)methyl]amino}-1-oxopropan-2-yl)phenyl trifluoromethanesulfonate;
4-(1-{[3-[3-methoxybenzyl(methyl)amino]propyl]amino-1-oxopropan-2-yl)phenyl trifluaramethanesulfonate;
4-[3-(3,4-dimethylphenoxy)-2-hydroxypropyl]amino-1-oxopropan-2-yl)phenyl trifluoromethanesulfonate;
2-(3-{[3-methoxy-5-(trifluoromethyl)phenyl]amino}pheyl)-*N-*(3-ethoxypropyl) propanamide;
2-(3-{[3-methoxy-5-(trifluoromethyl)phenyl]amino}phenyl)-*N*-(1*H*-pyrrol-1-yl) propanamide;
*N*'-{2-[3-(3-methoxy-5-(trifluoromethyl)phenylamino)phenyl]propanoyl}furan-2-carbohydrazide;
2-(3-{[3-methoxy-5-(trifluoromethyl)phenyl]amino}phenyl)-*N*-(pyrimidin-4-yl) propanamide;
*N*-ethyl-2-(3-{[3-methoxy-5-(trifluoromethyl)phenyl]amino}phenyl)propanamide
2-{3-[(3-methoxy-5-(trifluoromethyl)phenyl)amino]phenyl}-*N*-[2-(benzylamino)ethyl]propanamide;
*N*-(2-amino-2-methylpropyl)-2-[3-1[3-methoxy-5-(trifluoromethyl)phenyl] amino}phenyl]propanamide;
*N*-(2-aminocyclohexyl)-2-[3-([3-methoxy-5-(trifluoromethyl)phenyl] amino}phenyl]propanamide;
methyl 3-[(tert-butoxycarbonyl)amino]-2-[4-(naphthalen-1-yloxyphenyl)propanoyl] aminopropanoate;
*N*-[2-(benzylamino)ethyl]-2-[4-(naphthalen-1-yloxy)phenyl]propanamide;
*N*-[3-(dimethylamino)propyl]-2-[4-(naphthaten-1-yloxy)phenyl]propanamide;
*N*-[3-(cyclohexylamino)propyl]-2-[4-(naphthalen-1-yloxy)phenyl]propanamide;
2-[4-(naphthalen-1-yloxy)phenyl]-*N*-(4*H*-1,2,4-triazol-4-yl)propanamide;
2-[4-(naphthalen-1-yloxy)phenyl]-*N*-[2-(1-methylpyrrolidin-2-yl)ethyl]propanamide;
*N*-[2-(acetylamino)ethyl]-2-[4-(naphthalen-1-yloxy)phenyl]propanamide;
2-[4-(naphthalen-1-yloxy)phenyl]-*N*-[2-(morpholin-4-yl)ethyl]propanamide;
2-[4-(piperidin-1-yl)phenyl]-*N*-(pyrimidin-4-yl)propanamide;
*N*-{2-[1-(pyridin-4-yl)piperidin-4-yl]ethyl}-2-(4-{[2-(1*H*-pyrrol-1-yl)phenyl]amino}phenyl)propanamide;
2-{4-[(4-fluorophenyl)amino]pheny1}-*N*-(pyridin-4-yl)propanamide;
2-[4-(4-fluorophenoxy)phenyl]-*N*-(pyrimidin-4-yl)propanamide;
2-[3-(naphthalen-1-yloxy)phenyl]-*N*-(pyridin-4-yl)propanamide;
2-{3-[(4-fluorophenyl)amino]phenyl}-*N*-(pyridin-4yl)propanamide;
2-[4-(4-fluorophenoxy)phenyl]-*N*-(pyrazin-2-yl)propanamide;
2-{3-[(2,2-difluoro-1,3-benzodioxol-5-yl)amino]phenyl}propanamide;
2-[4-(piperidin-1-yl)phenyl]-*N*-(pyrazin-2-yl)propanamide;
2-(4-{[(4-chlorophenyl)sulfonyl]amino}phenyl)-*N*-(4*H*-1,2,4-triazol-4-yl)propanamide;
2-{4-[(2,2-difluoro-1,3-benzodioxol-5-yl)amino]phenyl}propanamide;
*N*-(pyridin-4-yl)-2-[4-(quinolin-3-ylamino)phenyl]propanamide;
4-{1-[(3,5-dichloro-2-sulfamoylphenyl)amino]-1-oxopropan-2-yl}phenyl-2-chlorobenzenesulfonate;
2-[4-{[2-(1H-pyrrol-1-yl)phenyl]-N-(pyridin-4-yl)propanamide.

9. Compounds as claimed in claims 1 to 8 for use as inhibitors of Bradykinin B1 receptor;

10. Compounds as claimed in claims 1 to 9 for use in the treatment and prevention of diseases and conditions mediated by Bradikinin B1 receptor pathway.

11. Compounds as claimed in claim 10 for use in the prevention and/or treatment of pain and/or inflammation.

12. Compounds as claimed in claim 10 for use in the prevention and/or treatment of visceral pain neuropathic pain, central pain syndromes, postsurgical pain syndromes, bone and joint pain, repetitive motion pain, dental pain, cancer pain, myofascial pain, perioperative pain, chronic pain, dysmenorrea, pain associated with angina and inflammatory pain.

13. Compounds as claimed in claim 10, for the prevention and/or treatment of pain associated to pancreatitis, cystitis, renal colics, post herpetic neuralgia, nerve injury, osteoarthritis, muscular injury, fibromyalgia, rheumatoid arthritis, rheumatic disease and gout.

14. Compounds as claimed in claim 10, for the prevention and/or treatment of hyperreactive airways and inflammatory events associated with airway disease.

15. Compounds as claimed in claim 10 for the prevention and/or treatment of asthma, bronchoconstriction, occupational asthma, viral- or bacterial-exacerbation of asthma, non-allergic asthmas, "wheezy-infant syndrome", chronic obstructive pulmonary disease and pneumoconiosis.

16. Compounds as claimed in claim 15 wherein said chronic obstructive pulmonary disease comprises eznphysema, ARDS, bronchitis, pneumonia, allergic and vasomotor rhinitis.

17. Compounds as claimed in claim 15 wherein said pneumoconiosis comprises aluminosis, anthracosis, asbestosis, chalicosis, ptilosis, siderosis, tabacosis and byssinosis

18. Compounds as claimed in claim 10, for the treatment of inflammatory bowel diseases inflammatory skin disorders, edema resulting from burns, sprains and fractures, cerebral edema, angioedema, diabetic vasculopathy, diabetic neuropathy, diabetic retinopathy, diabetic symptoms associated with insulitis.

19. Compounds as claimed in claim 18 wherein said inflammatory bowel disease comprises Crohn's disease and ulcerative colitis and uveitis.

20. Compounds as claimed in claim 18 wherein said inflammatory skin disorders are psoriasis and eczema.

21. Compounds as claimed in claims 10 for use in the prevention and/or treatment of a disease selected from liver disease, multiple sclerosis, cardiovascular disease, neurodegenerative diseases, epilepsy, septic shock, headache, migraine, closed head trauma, cancer, sepsis, gingivitis, osteoporosis, benign hyperplasia and hyperactive bladder

22. Pharmaceutical compositions comprising a compound as claimed in claims 1 to 21 in admixture with pharmaceutically acceptable excipients and/or diluents.
